(19) Europäisches Patentamt
European Patent Office
Office européen des brevets

(11) EP 2 393 768 B1

(12) **EUROPÄISCHE PATENTSCHRIFT**

(45) Veröffentlichungstag und Bekanntmachung des Hinweises auf die Patenterteilung:
**13.05.2015 Patentblatt 2015/20**

(21) Anmeldenummer: **10701238.7**

(22) Anmeldetag: **25.01.2010**

(51) Int Cl.:
***C07C 45/38*** *(2006.01)* ***C07C 45/39*** *(2006.01)*
***C07D 213/48*** *(2006.01)* ***C07D 307/48*** *(2006.01)*
***C07D 493/04*** *(2006.01)* ***C07C 47/02*** *(2006.01)*
***C07C 49/433*** *(2006.01)* ***C07C 49/403*** *(2006.01)*

(86) Internationale Anmeldenummer:
**PCT/EP2010/050772**

(87) Internationale Veröffentlichungsnummer:
**WO 2010/089213 (12.08.2010 Gazette 2010/32)**

(54) **VERFAHREN ZUR HERSTELLUNG VON ALDEHYDEN UND KETONEN AUS PRIMÄREN UND SEKUNDÄREN ALKOHOLEN**

METHOD FOR PRODUCING ALDEHYDES AND KETONES FROM PRIMARY AND SECONDARY ALCOHOLS

PROCÉDÉ POUR PRÉPARER DES ALDÉHYDES ET DES CÉTONES À PARTIR D'ALCOOLS PRIMAIRES ET SECONDAIRES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **06.02.2009 DE 102009000662**

(43) Veröffentlichungstag der Anmeldung:
**14.12.2011 Patentblatt 2011/50**

(73) Patentinhaber: **Evonik Degussa GmbH**
**45128 Essen (DE)**

(72) Erfinder:
- **DINGERDISSEN, Uwe**
  **64342 Seeheim (DE)**
- **PFEFFER, Jan**
  **45355 Essen (DE)**
- **TACKE, Thomas**
  **63755 Alzenau (DE)**
- **HAAS, Thomas**
  **48161 Münster (DE)**
- **SCHMIDT, Harald**
  **45481 Mülheim an der Ruhr (DE)**
- **KLASOVSKY, Florian**
  **45721 Haltern am See (DE)**
- **SHELDON, Roger**
  **NL-6997 Hoog-Kappel (NL)**
- **JANSSEN, Michiel**
  **NL-2497 DR Den Haag (NL)**
- **VOLLAND, Michael**
  **48249 Dülmen (DE)**
- **RIMBACH, Michael**
  **44649 Herne (DE)**
- **RINKER, Stefanie**
  **46569 Hünxe (DE)**

(56) Entgegenhaltungen:
**WO-A1-2009/145323** **US-A- 5 136 103**
**US-A- 5 336 811**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 393 768 B1

## Beschreibung

Die vorliegende Erfindung beschreibt ein Verfahren zur Herstellung von Aldehyden und Ketonen aus günstig zugänglichen primären und sekundären Alkoholen durch Oxidation mit Luftsauerstoff oder reinem Sauerstoff mittels eines Katalysatorsystems bestehend aus einem freien Nitroxylradikalderivat.

Stabile Nitroxylradikalderivate wurden erstmals bei Hoffmann (A.K. Hoffmann, A.T. Henderson, J. Am. Chem. Soc. 83 (1961)4671) und Lebeder (O.L. Lebeder, S.N. Kazarnovskii, Zh. Obshch. Khim. 30 (1960) 1631; O.L. Lebeder, S.N. Kazarnovskii, CA 55 (1961) 1473a.) beschrieben. Ihre erste Verwendung fanden sie als Radikalfänger. Erst kürzlich wurde ihre Bedeutung als Katalysatoren für die Oxidation von Alkoholen entdeckt (z. B.: J.M. Bobbitt, C.L. Flores, Heterocycles 27 (1988) 509 oder A.E.J. de Nooy, A.C. Besemer, H. van Bekkum, Synthesis (1996) 1153.).

Nachteilig wirkt sich bei dieser Art der Katalyse aus, dass der zur Oxidation erforderliche Sauerstoff häufig aus teuren Sauerstoffquellen generiert wird. Berichtet wird z. B. über den Einsatz von Hypochlorit, Chlorperbenzoesäure, Peroxomonoschwefelsäure, Perjodsäure oder Trichloroisocyanursäure (z. B. L. Anelli, C. Biffi, F. Montanari, S. Quici, J. Org. Chem. 52 (1987) 2559; J.A. Cella, J.A. Kelley, E.F. Kenehan, J. Org. Chem. 40 (1975) 1850; S.D. Rychovsky, R. Vaidyanathan, J. Org. Chem. 64 (1999) 310.; Bolm, Carsten; Magnus, Angelika S.; Hildebrand, Jens P. Organic Letters (2000), 2(8),1173-1175.; S.S. Kim, K. Nehru, Synletter (2002) 616.; De Luca, Lidia; Giacomelli, Giampaolo; Porcheddu, Andrea. Organic Letters (2001), 3(19), 3041-3043.). Zudem enthalten viele der ausgeführten Reagenzien Halogene (insbesondere Chlor, Brom, Jod), die unter den Reaktionsbedingungen korrosiv wirken können und oft zu unerwünschten Nebenreaktionen führen.

Die Oxidation der Alkohole mittels Sauerstoff unter Verwendung von Nitroxylradikalderivaten gelingt durch Zugabe von Übergangsmetallen, wie z. B. Kobalt, Kupfer, Wolfram, Ruthenium, Mangan und Eisen (z. B. Sheldon, Roger A.; Arends, Isabel W. C. E. Journal of Molecular Catalysis A: Chemical (2006), 251(1-2), 200-214.; Minisci, Francesco; Punta, Carlo; Recupero, Francesco. Journal of Molecular Catalysis A: Chemical (2006), 251(1-2), 129-149.). Nachteilig bei dieser Methode ist die häufig schwierige Abtrennung der Übergangsmetallsalze und deren toxische Eigenschaften.

Augustine (US 7,030,279) beschreibt allgemein die Oxidation von primären oder sekundären Alkoholen mit Sauerstoff als Oxidans zu den korrespondierenden Aldehyden und Ketonen mittels eines Katalysatorsystems bestehend aus einem freien Nitroxylradikalderivat, einer Nitratquelle, einer Bromquelle und einer Carbonsäure, wobei die Carbonsäure in allen Fällen Essigsäure ist.

Xinquan Hu et al. beschreiben in J. AM. CHEM. SOC. 2004, 126, 4112-4113 die Alkohol-Oxidation mittels Nitroxylradikalderivat und Sauerstoff, wobei die Nitratquelle durch eine Nitritquelle ersetzt wurde. Hierbei wird allerdings explizit ausgeführt, dass auf den Einsatz einer Bromquelle nicht verzichtet werden kann. Das aufgeführte Lösungsmittel ist in den beschriebenen Beispielen Dichlormethan.

Auch mit der Methode von Augustine und Xinquan Hu kann auf den Einsatz von Halogenquellen nicht verzichtet werden.

Die halogenfreie und übergangsmetallfreie Katalyse mittels Nitroxylradikalderivaten und Sauerstoff ist bei Fried (US 5,136,103 und US 5,155,280) beschrieben, wobei dabei als weiteres Reagenz Salpetersäure zum Einsatz kommt. Hierbei werden allerdings für die Ketone nur Ausbeuten unter 90 % und im Falle der Aldehyde nur Umsätze zwischen 24 und 72 % gefunden. Viel eher gelingt es mit dieser Methode die Carbonsäuren zu synthetisieren (US 5,239,116). Erst wenn die Salpetersäure als stöchiometrisches Reagenz eingesetzt wird, d.h. wenn auf Sauerstoff als günstiges Oxidans verzichtet wird (US 5,155,279), werden Umsätze von 42-84 %, bei Selektivitäten von 69-81 % gefunden.

US 5,336,811 beschreibt einen Prozess zur Herstellung eines Aldehyds durch Reaktion des korrespondierenden Arylmethanols mit einem stabilen radikalischen Nitroxid in Gegenwart einer $No_x$-generierenden Verbindung bei einer Temperatur im Bereich von etwa 0 °C bis etwa 100 °C und anschließende Abtrennung des Aldehyds.

Die Verfahren gemäß dem Stand der Technik haben den Nachteil, dass sie häufig aufgrund der geringen Ausbeuten und vielen Verfahrensschritte oder dem stöchiometrischem Einsatz von teuren, korrosiven und giftigen Reagenzien für eine großtechnische Herstellung von Aldehyden und Ketonen ungeeignet sind. Insbesondere die bei der Nitroxylradikal katalysierten Umsetzung von Alkoholen benötigten Zusätze von Halogenquellen in Verbindung mit Carbonsäuren, aber auch in organischen Lösungsmitteln, stellen äußerst korrosive Systeme dar, die eine Überführung in den technischen Maßstab erschweren. Alternativ werden häufig giftige und aufwendig zu entfernende Übergangsmetalle als Katalysatorbestandteil eingesetzt.

Es war daher die Aufgabe der vorliegenden Erfindung, ein Verfahren zur Herstellung von Aldehyden und Ketonen bereitzustellen, wobei als Edukt primäre oder sekundäre Alkohole eingesetzt werden können, und das so gestaltet ist, dass auf die Zugabe von Halogenquellen, insbesondere Bromquellen und die Verwendung von Übergangsmetallen verzichtet werden kann.

Überraschenderweise wurde ein Verfahren zur Herstellung von Aldehyden und Ketonen gefunden, wobei als Edukt primäre und sekundäre Alkohole eingesetzt werden können, und das genau die oben genannten Nachteile des Standes der Technik überwindet.

Gegenstand der vorliegenden Erfindung ist demgemäß ein Verfahren zur Herstellung von Aldehyden und Ketonen umfassend die Oxidation von primären oder sekundären Alkoholen mit einem sauerstoffhaltigen Gas in Gegenwart einer Katalysatorzusammensetzung umfassend mindestens ein Nitroxylradikal, eine oder mehrere NO-Quellen und mindestens eine oder mehrere Carbonsäuren oder deren Anhydride oder eine oder mehrere Carbonsäuren oder deren Anhydride und Mineralsäuren oder deren Anhydride, ggf. in Gegenwart eines oder mehrerer Lösungsmittel, dadurch gekennzeichnet, dass die eingesetzten primären und sekundären Alkohole einen Wert für den dekadischen Logarithmus des n-Oktanol-Wasser-Verteilungskoeffizienten (logP) von weniger als 2 aufweisen und dass die Aldehyde und Ketone vorzugsweise mit einer Ausbeute von mehr als 92 %, bezogen auf den eingesetzten Alkohol erhalten werden.

Das erfindungsgemäße Verfahren hat den Vorteil, dass der Alkohol mit einer milden Methode in Gegenwart von Nitroxylradikalen in einem einzigen Verfahrensschritt oxidiert wird. Mit dieser milden Methode können überraschenderweise auch sterisch gehinderte Alkohole oxidiert werden, die mittels anderer Methoden nicht zugänglich sind. Bei dieser Oxidation gelingt es häufig nahezu quantitative Umsätze und Selektivitäten zu erzielen, d.h. die hohen Ausbeuten belegen die hohe Effizienz des erfindungsgemäßen Verfahrens. Die ist auf Grundlage des Standes der Technik völlig überraschend.

Insbesondere gelingt es korrosive Halogenquellen zu vermeiden, ebenso wie die Verwendung von Übergangsmetallen, und das Substrat, das die NO-Quelle darstellt, den jeweiligen Prozessanforderungen durch Variation so anzupassen, dass keine schwierig und damit energieintensiv aufzuarbeitenden Salzfrachten entstehen. Von besonderem Vorteil ist aber, dass das Verfahren ohne Zusatz von externen Halogenquellen, also halogenfrei erfolgt. Halogenquellen im Sinne der vorliegenden Erfindung meint alle halogenhaltigen Verbindungen, die Halogene in elementarer Form oder halogenhaltige Ionen in beliebiger Oxidationsstufe freisetzen können. Ein Zusatz halogenhaltiger Verbindungen, wie Chlorite oder bromhaltige Verbindung wie *N*-Bromsuccinimid, *N*-Bromphthalimid, Tetrabutylammoniumbromid oder anorganische Salze, wie beispielsweise $NH_4Br$, Alkali- oder Erdalkalibromide zur Durchführung der Oxidation, kann, anders als im Stand der Technik, also entfallen.

Daraus ergibt sich, dass auch das erhaltene Verfahrensprodukt weitestgehend halogenfrei ist, wobei halogenfrei im Sinne der vorliegenden Erfindung meint, dass kein Halogen aus einer externen Halogenquelle stammt.

In dem Verfahrensschritt des erfindungsgemäßen Verfahrens werden primäre und sekundäre Alkohole eingesetzt, wobei auch der Einsatz mehrfunktioneller Alkohole und Poly-Alkohole möglich ist. Ein besonderer Vorteil ist, dass mit dem erfindungsgemäßen Verfahren auch die Umsetzung von sterisch anspruchsvollen Alkoholen zu den Alkoholen bzw. Ketonen gelingt. Grundbedingung für die einsetzbaren Alkohole ist, dass sie einen Wert für den dekadischen Logarithmus des n-Oktanol-Wasser-Verteilungskoeffizienten (log P) von weniger als 2,4, vorzugsweise weniger als 2 aufweisen.

Der n-Oktanol-Wasser-Verteilungskoeffizient $K_{ow}$ oder P ist ein dimensionsloser Verteilungskoeffizient, der das Verhältnis der Konzentrationen einer Chemikalie in einem Zweiphasensystem aus 1-Oktanol und Wasser angibt (siehe J. Sangster, Octanol-Water Partition Coefficients: Fundamentals and Physical Chemistry, Vol. 2 of Wiley Series in Solution Chemistry, John Wiley & Sons, Chichester, 1997**).**

Der $K_{ow}$- bzw. P-Wert bezieht sich immer nur auf eine Spezies einer Chemikalie und wird durch folgende Gleichung dargestellt:

$$K_{OW} = P = \frac{c_o^{S_i}}{c_w^{S_i}}$$

mit:

$c_o^{S_i}$ = Konzentration der Spezies i einer Chemikalie in der Oktanol-reichen Phase

$c_w^{S_i}$ = Konzentration der Spezies i einer Chemikalie in der wasserreichen Phase

P wird in der Regel in der Form des dekadischen Logarithmus als log P (auch log $P_{ow}$ bzw. seltener log pOW) angegeben:

Der $K_{ow}$-Wert ist ein Modellmaß für das Verhältnis zwischen Lipophilie (Fettlöslichkeit) und Hydrophilie (Wasserlöslichkeit) einer Substanz. Die Erwartung ist, mit Hilfe des Verteilungskoeffizienten eines Stoffes im System Oktanol-Wasser auch die Verteilungskoeffizienten dieses Stoffes in anderen Systemen mit einer wässrigen Phase abschätzen zu können. $K_{ow}$ ist größer als eins, wenn eine Substanz besser in fettähnlichen Lösungsmitteln wie n-Oktanol löslich ist, kleiner als eins wenn sie besser in Wasser löslich ist. Entsprechend ist Log P positiv für lipophile und negativ für hydrophile Substanzen. Da nicht für alle Chemikalien der KOW gemessen werden kann, gibt es verschiedenste Modelle für die Vorhersage, z. B. durch Quantitative Struktur-Aktivitäts-Beziehungen (QSAR) oder durch Linear Free Energy Relationships (LFER), beispielsweise beschrieben in Eugene Kellogg G, Abraham DJ: Hydrophobicity: is LogP(o/w) more than the sum of its parts?. Eur J Med Chem. 2000 Jul-Aug;35(7-8):651-61 oder Gudrun Wienke, "Messung und Vorausberechnung von n-Octanol/Wasser-Verteilungskoeffizienten", Doktorarbeit, Univ. Oldenburg, 1-172, 1993.

Im Rahmen der vorliegenden Erfindung wird log P nach der Methode von Advanced Chemistry Development Inc., Toronto mittels des Programmmoduls ACD/LogP DB bestimmt.

Beispiele entsprechender Alkohole sind die niederkettigen aliphatischen Alkohole wie z. B. Ethanol, Ethylenglycol, Glycerin aber auch höhermolekulare Systeme wie die Zuckeralkohole, beispielsweise Isosorbit, Isommanit und deren Derivate, oder Polyole, wie z. B. Polyethylenglycole. Die Oxidation des erfindungsgemäßen Verfahrens wird mit einer Katalysatorzusammensetzung in Abwesenheit von Übergangsmetallen durchgeführt.

Wesentlicher Bestandteil der im erfindungsgemäßen Verfahren eingesetzten Katalysatorzusammensetzung sind die Nitroxylradikale. Im Rahmen dieser Erfindung werden unter Nitroxylradikalen Verbindungen verstanden, die die Atomgruppierung

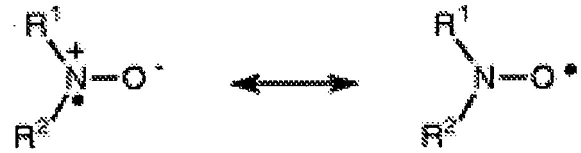

enthalten und die bei Raumtemperatur in Gegenwart von Sauerstoff mindestens eine Woche stabil sind. Diese Nitroxylradikale weisen am α-C-Atom benachbart zum Stickstoffatom keine Wasserstoffatome auf.

In dem erfindungsgemäßen Verfahren werden als Nitroxylradikale in der Katalysatorzusammensetzung bevorzugt Verbindungen gemäß der Struktur (I) und/oder salzartige Verbindungen gemäß der der Struktur **(II)** eingesetzt:

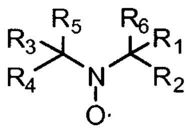

(I)

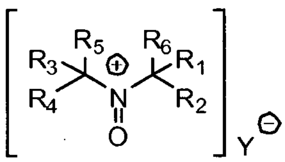

(II)

mit:

$R^1$, $R^2$, $R^3$, $R^4$, $R^5$ und $R^6$ = ($C_1$-$C_{10}$)-Alkyl-, ($C_1$-$C_{10}$)-Alkenyl-, ($C_1$-$C_{10}$)-Alkoxy-, ($C_6$-$C_{18}$)-Aryl-, ($C_7$-$C_{19}$)-Aralkyl-, ($C_6$-$C_{18}$)-Aryl-($C_1$-$C_8$)-Alkyl- oder ($C_3$-$C_{18}$)-Heteroarylgruppe,
wobei die Substituenten vom Typ $R^1$, $R^2$, $R^3$, $R^4$, $R^5$ und $R^6$ gleich oder verschieden sind und die Substituenten vom Typ $R^5$ und $R^6$ zusammen eine ($C_1$-$C_4$)-Alkylenbrücke bilden können, welche gesättigt oder ungesättigt, unsubstituiert oder substituiert sein kann, insbesondere mit einem oder mehreren Substituenten, ausgewählt aus $R^1$, $C_1$-$C_8$-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe. In der Struktur **(II)** ist $Y^-$ ein beliebiges halogenfreies Anion.

In dem erfindungsgemäßen Verfahren kann sowohl ein Nitroxylradikal, es kann aber auch eine Mischung aus verschiedenen Nitroxylradikalen eingesetzt werden.

Vorzugsweise werden in dem erfindungsgemäßen Verfahren als Nitroxylradikale 2,2,6,6-Tetramethylpiperidin-1-oxyl (TEMPO) und/oder die an Position 4 des Heterocyclus substituierten Derivate des 2,2,6,6-Tetramethylpiperidin-1-oxyls eingesetzt, wobei die Derivate einen oder mehrere Substituenten ausgewählt aus $R^1$, $C_1$-$C_8$-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen aufweisen, worin $R^1$ eine ($C_1$-$C_{10}$)-Alkyl-, ($C_1$-$C_{10}$)-Alkenyl-, ($C_1$-$C_{10}$)-Alkoxy-, ($C_6$-$C_{18}$)-Aryl-, ($C_7$-$C_{19}$)-Aralkyl-, ($C_6$-$C_{18}$)-Aryl-($C_1$-$C_8$)-Alkyl- oder ($C_3$-$C_{18}$)-Heteroarylgruppe bedeutet. Beispiele entsprechender Verbindungen sind4-Methoxy-2,2,6,6-tetramethylpiperidin-1-oxyl (4-MeO-TEMPO), 4-Oxo-2,2,6,6-tetramethylpiperidin-1-oxyl (4-oxo-TEMPO), 4-Hydroxy-2,2,6,6-tetramethylpiperidin-1-oxyl (4-hydroxy-TEMPO), 4-Benzoyloxy-2,2,6,6-tetramethylpiperidin-1-oxyl (BnO-TEMPO), 4-Acetamido-2,2,6,6-tetramethylpiperidin-1-oxyl, 4-Acetamino-2,2,6,6-tetramethylpiperidin-1-oxyl (AA-TEMPO), 4-Amino-2,2,6,6-tetramethylpiperidin-1-oxyl, N,N-Dimethylamino-2,2,6,6-tetramethyl-piperidin-1-oxyl (NNDMA-TEMPO), 3,6-Dihydro-2,2,6,6-tetramethyl-1(2H)-pyridinyl-oxyl (DH-TEMPO) oder Bis-(2,2,6,6-tetramethylpiperidin-1-oxyl-4-yl) sebacat, wobei die genannten Beispiele einen oder mehrere Substituenten, ausgewählt aus $R^1$, $C_1$-$C_8$-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe, aufweisen können.

Das Tetramethylpiperidin-N-oxyl-Strukturfragment gemäß den Strukturen **(I)** oder **(II)** kann auch ein Bestandteil eines größeren Makromoleküls, eines Oligomers oder auch einer Polymerstruktur sein. Ein Beispiel für ein solches Nitroxylradikal zeigt die Struktur **(III):**

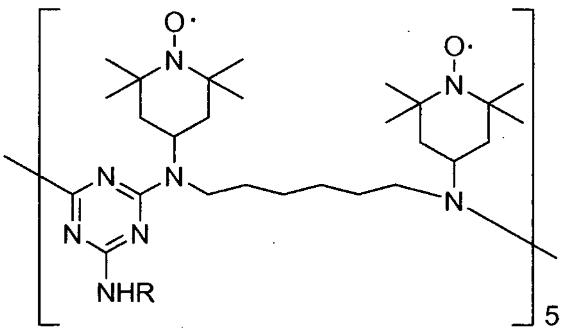

**(III)**

Die Nitroxylradikale können auch in heterogener Form in dem erfindungsgemäßen Verfahren eingesetzt werden, dies bedeutet dass die Nitroxylradikale auf einen Träger, beispielsweise Aluminiumoxid, Siliziumdioxid, Titandioxid oder Zirkoniumdioxid, aufgetragen sind. Als Trägermaterial für das Nitroxylradikal sind auch Polymere, Verbundstoffe oder Kohlenstoff einsetzbar.

Bevorzugt werden in dem erfindungsgemäßen Verfahren die oben genannten Verbindungen AA-TEMPO, 4-Hydroxy-TEMPO, TEMPO und 4-Oxo-TEMPO als Nitroxylradikale eingesetzt. Besonders bevorzugt werden AA-TEMPO, 4-Hydroxy-TEMPO und TEMPO, insbesondere das AA-TEMPO, eingesetzt.

In dem erfindungsgemäßen Verfahren beträgt der Anteil an Nitroxylradikal vorzugsweise 0,001 bis 10 mol-%, bevorzugt 0,01 bis 5 mol-% und besonders bevorzugt 0,1 bis 2 mol-%, bezogen auf die Menge des eingesetzten Alkohols.

Weiterhin enthält die in dem erfindungsgemäßen Verfahren eingesetzte Katalysatorzusammensetzung mindestens eine NO-Quelle. Als NO-Quelle können in dem erfindungsgemäßen Verfahrens Ammoniumnitrat oder -nitrit, oder Alkali- oder Erdalkalinitrate oder -nitrite, wie beispielsweise Magnesiumnitrat, oder Natriumnitrit eingesetzt werden. Zudem können in Ergänzung zu den Nitraten oder Nitriten oder als Ersatz für die Nitrate oder Nitrite stickoxidhaltige Gase wie $N_2O$, NO, $N_2O_3$. $NO_2$, $N_2O_4$,und $N_2O_5$. eingesetzt werden. Als NO-Quelle können auch Mischungen verschiedener, der oben genannten NO-Quellen eingesetzt werden. In dem erfindungsgemäßen Verfahren beträgt der Anteil der eingesetzten NO-Quelle(n) 0,001 bis 10 mol-%, bevorzugt von 0,01 bis 5 mol-% und ganz besonders bevorzugt von 0,1 bis 2 mol-%, bezogen auf die Menge an eingesetztem Alkohol.

Weiterhin enthält die in dem erfindungsgemäßen Verfahren eingesetzte Katalysatorzusammensetzung mindestens eine oder mehrere Carbonsäuren oder deren Anhydride oder eine oder mehrere Carbonsäuren oder deren Anhydride und Mineralsäuren oder deren Anhydride. Als Carbonsäure oder Carbonsäureanhydrid wird in dem erfindungsgemäßen Verfahren, vorzugsweise Essigsäure oder Essigsäureanhydrid, Propionsäure oder eine andere Carbonsäure oder ein anderes Anhydrid, die sich in dem Reaktionsgemisch löst, eingesetzt. Bevorzugt wird in dem erfindungsgemäßen Verfahren Essigsäure eingesetzt. Es können auch Mischungen verschiedener geeigneter Carbonsäuren oder Lösungen von Carbonsäuren in einem geeigneten Lösemittel eingesetzt werden. Bevorzugt werden von 0,1 bis 200 mol-% und besonders bevorzugt von 10 bis 50 mol-% an Carbonsäure bezogen auf die Menge an eingesetztem Alkohol eingesetzt.

Im Sinne der vorliegenden Erfindung wird der Terminus "Mineralsäuren" als Sammelbezeichnung für alle anorganischen Säuren verwendet. Geeignete Mineralsäuren sind beispielsweise $H_2CO_3$, $H_3PO_4$, $HNO_3$, $HNO_2$, $H_2SO_4$, $H_2SO_3$, $H_3BO_3$, deren Anhydride oder Mischungen hieraus.

Als Oxidationsmittel wird in dem erfindungsgemäßen Verfahren ein sauerstoffhaltiges Gas eingesetzt. Als sauerstoffhaltiges Gas kann reiner Sauerstoff eingesetzt werden, es können jedoch auch Mischungen von Sauerstoff mit einem Inertgas oder Luft oder einem an der Reaktion beteiligten Gas eingesetzt werden. Geeignete Inertgase sind beispielsweise Stickstoff, Kohlendioxyd, Helium oder Argon. Als an der Reaktion beteiligte Gase können beispielsweise Stickoxide eingesetzt werden, die bereits vorab bei der Beschreibung der NO-Quellen genannt wurden. Der Partialdruck

des Sauerstoffs beträgt bevorzugt 0,1 bis 100 bar, besonders bevorzugt 0,2 bis 50 bar.

Die Durchführung des erfindungsgemäßen Verfahrens kann in einem Lösungsmittel oder ohne Einsatz eines Lösungsmittels erfolgen:

In einer besonderen Ausführungsform des erfindungsgemäßen Verfahrens wird der Verfahrensschritt in Gegenwart eines Lösemittels durchgeführt. Hierbei werden vorzugsweise polare Lösemittel eingesetzt, insbesondere polare organische Lösungsmittel. Bevorzugt wird als Lösungsmittel Acetonitril, Tetrahydrofuran, Ethylacetat, Aceton, Diethylether, Methyl-tert.-butylether, tertiäre Alkohole wie tert.-Amlyalkohol, tert.-Butylalkohol, Ester der Kohlensäure wie Dimethylcarbonat, Diethylcarbonat, Kohlenwasserstoffe, oder eine Mischung dieser Lösungsmittel eingesetzt. Es werden vorzugsweise 0,1 bis 70 Vol-%, bevorzugt 0,5 bis 60 Vol-% und ganz besonders bevorzugt 1 bis 50 Vol-% an Lösungsmittel, bezogen auf die eingesetzte Menge an Alkohol, eingesetzt.

In einer weiteren bevorzugten Ausführungsform des erfindungsgemäßen Verfahrens wird in dem Verfahrensschritt kein zusätzliches Lösungsmittel eingesetzt. In diesem Fall dient die Carbonsäure oder die mineralische Säure nicht nur als Komponente der Katalysatorzusammensetzung sondern auch als Lösungsmittel, um die Reaktionsmischung homogen zu halten. Dies hat den Vorteil, dass auf Einsatz brennbarer und ggf. gesundheitsschädlicher Lösungsmittel verzichtet werden kann, bzw. eine gesonderte Abtrennung des Lösungsmittels entfallen kann.

Im Folgenden wird die Durchführung des erfindungsgemäßen Verfahrens erläutert. Die Oxidation des erfindungsgemäßen Verfahrens wird vorzugsweise bei einer Temperatur von 0 bis 100 °C, oder am Siedepunkt des Lösungsmittels durchgeführt.

Der Gesamtdruck bei der Oxidation im erfindungsgemäßen Verfahren beträgt vorzugsweise 1 bis 300 bar, bevorzugt 1 bis 50 bar.

Das Verfahren kann sowohl als Batch-, Semi-Batch- oder als kontinuierliches Verfahren durchgeführt werden. Des Weiteren ist das erfindungsgemäße Verfahren an keinen bestimmten Reaktortyp gebunden, vielmehr kann der Verfahrensschritt in einem Rührkessel, in einem Rohrreaktor, in einer Kesselkaskade, in einem Mikroreaktor oder einer Kombination dieser Reaktortypen durchgeführt werden.

In einer Ausführungsform des erfindungsgemäßen Verfahrens wird zunächst der Alkohol in einem geeigneten Lösungsmittel gelöst oder suspendiert, anschließend wird die Katalysatorzusammensetzung dieser Lösung oder Suspension einzeln oder als Mischung zugegeben. Anschließend werden Druck und Temperatur eingestellt. Es ist jedoch auch möglich die Katalysatorzusammensetzung vorzulegen und die Lösung oder Suspension des Alkohols der Katalysatorzusammensetzung zuzugeben. Im Falle einer kontinuierlichen Verfahrensführung wird vorzugsweise der Alkohol mit den Reaktionsgasen in der Ausführung eines Rieselbettes zugeführt.

Besonders vorteilhaft in dem erfindungsgemäßen Verfahren ist es, wenn der Gehalt an Wasser in der Reaktionszusammensetzung möglichst gering ist, da hohe Anteile an Wasser die Ausbeuten verringern können. Darüber hinaus kann die Reaktion durch die Entfernung des Reaktionswassers aus dem Reaktionsgeschehen beschleunigt werden.

Um den Wasseranteil möglichst gering zu halten, bieten sich mehrere Verfahrensvarianten an. So können der Reaktionsmischung Wasser absorbierende Mittel zugesetzt werden, vorzugsweise Natriumsulfat, Calciumoxid und Molekularsiebe, z. B. Zeolithe. Darüber können auch konzentrierte stark hydratisierende Säuren oder Salze eingesetzt werden. Beispielsweise können die oben genannten Mineralsäuren bzw. deren Anhydride als Wasser bindende Säuren eingesetzt werden. Es ist weiterhin möglich, sehr stark Wasser komplexierende Lösungsmittel, wie z. B. Eisessig einzusetzen.

Darüber hinaus ist es auch möglich Lösungsmittel einzusetzen, die Wasser chemisch binden können. Vorzugsweise handelt es sich dabei um die Anhydride der im erfindungsgemäßen Verfahren eingesetzten Carbonsäuren. Somit wirken die Carbonsäureanhydride nicht nur als Lösungsmittel für die Reaktion, sondern gleichzeitig auch als Wasser bindendes Mittel. Auf diese Weise wird eine effiziente Reaktionsführung ermöglicht, die gleichzeitig zu hohen Ausbeuten führt, ein Effekt, der in dieser Weise aus dem Stand der Technik nicht bekannt ist.

Wahlweise kann das Reaktionswasser aus der Reaktionsmischung auch durch destillative Entfernung oder durch extraktive Entfernung aus dem Reaktionsgeschehen abgezogen werden.

Die Aufarbeitung der Reaktionsmischung geschieht in der Regel in Abhängigkeit von der Polarität der Zielmoleküle und der Löslichkeit der Nitroxylradikale. Im Falle gut wasserlöslicher Aldehyde und Ketone bietet sich folgende Vorgehensweise an:

a) Entfernen der Lösungsmittel und der Carbonsäure durch Destillation oder Extraktion
b) Extraktion und ggf. Rezyklierung der Nitroxylradikale
c) Entfernung der ggf. aus den NO-Quellen stammenden Salze durch Ionentauscher, Elektrodialyse, oder Ultrafiltration
d) Und/oder Reinigung des Produkts durch Kristallisation, Destillation, Extraktion und/oder chromatographische

Trennung.

Die genannten Verfahrensschritte können dabei allein oder in beliebiger Kombination zueinander durchgeführt werden.

Die nach dem erfindungsgemäßen Verfahren erhaltene Aldehyde und Ketone können in vorteilhafter Weise als Zwischenprodukt zur weiteren Umsetzung eingesetzt werden. So kann sie beispielsweise durch reduktive Aminierung mit Ammoniak, Hydroxylamin oder Hydrazin in die entsprechenden Amine überführt werden, die vorteilhaft als Rohstoff für die Kunststoffherstellung eingesetzt werden können.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

**Beispiele:**

***a) sterisch anspruchsvolle Alkohole in Eisessig:***

1. Oxidation von Isosorbit mit AA-Tempo / $O_2$ / Nitrat/Nitrit, bromfrei

Material:

| | | |
|---|---|---|
| Isosorbit (1,4:3,6-Dianhydro-D-sorbitol) | 100 mmol | → 73,07 g/L |
| AA-TEMPO | 5 mol-% | → 5,35 g/L |
| $NaNO_2$ | 2 mol-% | → 2,55 g/L |
| $Mg(NO_3)_2$ x $6H_2O$ | 2 mol-% | → 0,70 g/L |
| Essigsäure (100%) | 200 mL | |
| Dekan (interner Standard für Analytik) | 7g | → 35 g/L |
| **logP Isosorbit** | **-1,67** | |

Reaktionsbedingungen:

| | |
|---|---|
| Reaktionstemperatur: | T = 50 °C |
| Reaktionszeit: | t = 4 h |
| Sauerstoff: | 10 bar |
| Reaktorvolumen | 450 mL |

Durchführung:

Das Edukt, der Katalysator, die Salze und der interne Standard werden vorgelegt, in der Essigsäure (Zugabe der Essigsäure bis das Volumen von 200 ml erreicht ist) gelöst und in den Hochdruckreaktor überführt. Der Reaktor wird verschlossen und mit Stickstoff zweimal inertisiert. Anschließend wird die Lösung unter Rühren auf eine Temperatur von 50 °C aufgeheizt. Zum Start der Reaktion wird 10 bar Sauerstoff auf den Autoklaven aufgedrückt. Der Druck in dem Reaktor wird bei 10 bar konstant gehalten, wobei verbrauchter Sauerstoff durch eine geöffnete Sauerstoffversorgung bis zu einem Druck von 10 bar nachdosiert wird. Nach vier Stunden wird die Reaktion durch Inertisierung mit Stickstoff und Abkühlung des Systems abgebrochen.

Ergebnis:

Es wird ein Umsatz von größer 97% beobachtet bei einer Selektivität für das Diketon (2,6-Dioxabicyclo-(3.3.0)-octan-4,8-dion) von 100%.

Abbildung 1 zeigt die Entwicklung der Isosorbit-, Diketon- und Monoketon-Konzentrationen in Abhängigkeit

von der Zeit bei der Durchführung des erfindungsgemäßen Verfahrens. Dabei wurden die Isosorbit- und Diketon-Konzentrationen über Kalibrierkurven und dem internen Standard ausgewertet. Die Auswertung der Monoketon-Konzentrationen erfolgt über die Peakflächen.

2. Oxidation von Isomannit mit AA-Tempo / $O_2$ / Nitrit / Nitrat, bromfrei:

Material:

| | | |
|---|---|---|
| Isomannit (1,4:3,6-Dianhydro-D-mannitol) | 25 mmol | → 73,07 g/L |
| AA-TEMPO | 5 mol-% | → 5,35 g/L |
| $NaNO_2$ | 2 mol-% | → 2,55 g/L |
| $Mg(NO_3)$ x $6H_2O$ | 2 mol-% | → 0,70 g/L |
| Essigsäure (100%) | 50 mL | |
| Dekan (interner Standard für Analytik) | 1,75g | → 35 g/L |
| **logP Isomannit** | **-1,67** | |

Reaktionsbedingungen:

| | |
|---|---|
| Reaktionstemperatur: | T = 50 °C |
| Reaktionszeit: | t = 1,5 h |
| Sauerstoff: | 10 mL / min |

Durchführung:

Das Edukt, der Katalysator, die Salze und der interne Standard werden vorgelegt, in der Essigsäure (Zugabe der Essigsäure bis das Volumen von 50 ml erreicht ist) gelöst und in einen Vierhalskolben mit Rückflusskühler, Thermometer, Gaseinleitungsfritte und Septum zur Probennahme überführt. Anschließend wird die Lösung unter Rühren auf eine Temperatur von 50 °C aufgeheizt. Zum Start der Reaktion wird Sauerstoff mit einer Durchflussrate von 10 mL / min über die Gaseinleitungsfritte in die Reaktionslösung eingeleitet. Die Reaktion wird über einen Zeitraum von 1,5 h gefahren, wobei zur Aufnahme der Kinetik in Abständen von 15 min eine Probe entnommen wird.

Ergebnis:

Es wird ein Umsatz von 100% beobachtet bei einer Selektivität für das Diketon (2,6-Dioxabicyclo-(3.3.0)-octan-4,8-dion) von 100%.

Abbildung 2 zeigt die Entwicklung der Isomannit-, Diketon- und Monoketon-Konzentrationen in Abhängigkeit von der Zeit bei der Durchführung des erfindungsgemäßen Verfahrens. Dabei wurden die Isomannit- und Diketon-Konzentrationen über Kalibrierkurven und dem internen Standard ausgewertet. Die Auswertung der Monoketon-Konzentrationen erfolgt über die Peakflächen.

3. Oxidation von Isosorbit mit verschiedenen N-Oxy-Radikalen / $O_2$ / Nitrit / Nitrat bromfrei

Material:

| | | |
|---|---|---|
| Isosorbit (1,4:3,6-Dianhydro-D-sorbitol) | 10 mmol | → 73,07 g/L |
| N-Oxy-Radikal | 5 mol-% | |
| $NaNO_2$ | 2 mol-% | → 2,55 g/L |
| $Mg(NO_3)_2$ x $6H_2O$ | 2 mol-% | → 0,70 g/L |
| Essigsäure (100%) | 20 mL | |

(fortgesetzt)

| | | |
|---|---|---|
| Hexadekan (interner Standard für Analytik) | 0,05g | → 2,5 g/L |
| **logP Isosorbit** | **-1,67** | |

Reaktionsbedingungen:

| | |
|---|---|
| Reaktionstemperatur: | T = 50 °C |
| Reaktionszeit: | t = 1,5 h - 4,5 h |
| Sauerstoff: | 10 mL / min |

Durchführung:

Das Edukt, der Katalysator und die Salze werden in einem Dreihalskolben mit Rückflusskühler und Gaseinleitungsfritte vorgelegt, mit 20 ml Essigsäure versetzt und unter Rühren gelöst. Anschließend wird die Lösung unter Rühren auf eine Temperatur von 50 °C aufgeheizt. Zum Start der Reaktion wird Sauerstoff mit einer Durchflussrate von 20 mL / min über die Gaseinleitungsfritte in die Reaktionslösung eingeleitet. Die Reaktionen werden über einen Zeitraum von 1,5 h - 4,5 h gefahren, wobei zur Aufnahme der Kinetik in Abständen von 30 min eine Probe entnommen wird.

Ergebnis:

Nach dem oben beschriebenen Protokoll werden verschiedene N-Oxy-Radikale für die Reaktion eingesetzt. Die Reaktion wird je nach eingesetztem N-Oxy-Radikal über einen Zeitraum von 1,5 h - 4,5 h durchgeführt. Mit dem N-Oxy-Radikal AA-Tempo kann ein Umsatz von 100 % und eine Selektivität zum Diketon von 100% erzielt werden.

Abb. 3 zeigt die Ergebnisse der Vergleichsversuche hinsichtlich der Umsetzungen. Dargestellt wird die Oxidation von Isosorbit mit AA-Tempo (oben links), PIPO (oben rechts), Tempo (unten links) sowie ein Vergleich der Umsätze aller drei N-Oxy-Radikale in Abhängigkeit von der Zeit. Die Grafen zeigen die normalisierten Flächen der GC-Analytik und den Umsatz des Isosorbit in % ausgewertet mit Hexadekan als internen Standard.

4. Oxidation von Isosorbit mit AA-Tempo / $O_2$ / Nitrit, bromfrei

Material:

| | | |
|---|---|---|
| Isosorbit (1,4:3,6-Dianhydro-D-sorbitol) | 100 mmol | → 73,07 g/L |
| AA-TEMPO | 5 mol-% | → 5,35 g/L |
| $NaNO_2$ | 2,5 mol-% | g/L |
| Essigsäure (100%) | 200 mL | |
| Dekan (interner Standard für Analytik) | 7g | → 35 g/L |
| **logP Isosorbit** | **-1,67** | |

Reaktionsbedingungen:

| | |
|---|---|
| Reaktionstemperatur: | T = 50 °C |
| Reaktionszeit: | t = 4 h |
| Sauerstoff: | 10 bar |
| Reaktorvolumen | 450 mL |

Durchführung:

Das Edukt, der Katalysator, die Salze und der interne Standard werden vorgelegt, in der Essigsäure (Zugabe der Essigsäure bis das Volumen von 200 ml erreicht ist) gelöst und in den Hochdruckreaktor überführt. Der Reaktor

wird verschlossen und mit Stickstoff zweimal inertisiert. Anschließend wird die Lösung unter Rühren auf eine Temperatur von 50 °C aufgeheizt. Zum Start der Reaktion wird 10 bar Sauerstoff auf den Autoklaven aufgedrückt. Der Druck in dem Reaktor wird bei 10 bar konstant gehalten, wobei verbrauchter Sauerstoff durch eine geöffnete Sauerstoffversorgung bis zu einem Druck von 10 bar nachdosiert wird. Nach vier Stunden wird die Reaktion durch Inertisierung mit Stickstoff und Abkühlung des Systems abgebrochen.

Ergebnis:

Es wird ein Umsatz von größer 97% beobachtet bei einer Selektivität für das Diketon (2,6-Dioxabicyclo-(3.3.0)-octan-4,8-dion) von 100%.

5.Oxidation von Isosorbit mit Tempo /NaOCl / Bromid

(Vergleichsexperiment Chlorat-Methode)

Material:

| | |
|---|---|
| Isosorbit (1,4:3,6-Dianhydro-D-sorbitol) | → 20 g/L |
| Tempo | → 1,71 g/L |
| NaBr | → 11,30 g/L |
| NaOCl | → 81,5 g/L |
| Wasser | → 100 mL |
| **logP Isosorbit** | **-1,67** |

Reaktionsbedingungen:

| | |
|---|---|
| Reaktionstemperatur: | T = 2-5 °C |
| Reaktionszeit: | t = 2 h |

Durchführung:

2 g Isosorbit, 1127 mg Natriumbromid und 171 mg TEMPO werden in 100 ml Wasser suspendiert und in einem Eiswasserbad auf 0 °C gekühlt. Der kontinuierlich gemessene pH-Wert dieser Mischung wird durch Zugabe von 0,5 N Natronlauge exakt auf pH 10 eingestellt.

Der Versuch wird bei einem konstanten pH-Wert von 10 durchgeführt. Dieses wird durch die kontinuierliche Titration der entstehenden Säuren mit 0,5 N Natronlauge erreicht, wobei der pH-Wert mit einem automatischen Titrationssystem TITRINO, Firma Metrohm, Herisau (CH), konstant gehalten wird.

Ergebnis:

Die Reaktion führt zu geringen Mengen Monoketon und Diketon ist nicht zu erkennen.

6. Oxidation von Isosorbit mit AA-TEMPO / $O_2$ / Nitrit / $HNO_3$ (Vergleichsexperiment)

Material:

| | |
|---|---|
| Isosorbit (1,4:3,6-Dianhydro-D-sorbitol) | 10 mmol |
| AA-TEMPO | 5 mol-% |
| $NaNO_2$ | 2 mol-% |
| $HNO_3$ | 10 mol-% |

(fortgesetzt)

| | |
|---|---|
| Wasser | 20 mL |
| Hexadekan (interner Standard für Analytik) | 0,05g |

Reaktionsbedingungen:

| | |
|---|---|
| Reaktionstemperatur: | T=50°C |
| Reaktionszeit: | t=4h |
| Sauerstoff: | 20 mL / min |

Durchführung:

Das Edukt, der Katalysator, die Salze und die Salpetersäure werden in einem mit 10 ml Wasser gefülten Dreihalskolben mit Rückflusskühler und Gaseinleitungsfritte vorgelegt, auf 20 ml mit Wasser aufgefüllt und unter Rühren gelöst. Anschließend wird die Lösung unter Rühren auf eine Temperatur von 50°C aufgeheizt. Zum Start der Reaktion wird Sauerstoff mit einer Durchflussrate von 20 mL / min über die Gaseinleitungsfritte in die Reaktionslösung eingeleitet. Die Reaktionen werden über einen Zeitraum von 4 h gefahren.

Ergebnis:

Mit dem verwendeten Oxidationssystem konnte kein Umsatz und keine Reaktion sowohl zu Monoketon als auch zu Diketon beobachtet werden.

***b) sterisch anspruchslose Alkohole in Eisessig***

1. Oxidation von 2-Propanol mit AA-Tempo / $O_2$ / Salpetersäure, bromfrei

Material:

| | | |
|---|---|---|
| 2-Propanol | 200 mmol | → 119,34 g/L |
| AA-TEMPO | 3.5 mol-% | → 14,94 g/L |
| Salpetersäure (> 99.8 %) | 3.5 mol-% | → 4,53 g/L |
| Essigsäure (100%) | 100 mL | |
| **logP 2-Propanol** | **0.38** | |

Reaktionsbedingungen:

| | |
|---|---|
| Reaktionstemperatur: | T = 50 °C |
| Reaktionszeit: | t = 3 h |
| Sauerstoff: | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50 °C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine

geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 15 % beobachtet bei einer Selektivität für das Keton (Aceton) von 70%. Als Nebenprodukt kann Isopropylacetat in geringem Ausmaß detektiert werden.

Abbildung 4 zeigt die Entwicklung der 2-Propanol-, Aceton- und Isopropylacetat-Konzentrationen in Abhängigkeit von der Zeit bei der Durchführung des erfindungsgemäßen Verfahrens. Die Auswertung der Konzentrationen erfolgt über die Peakflächen.

2. Oxidation von 1-Propanol mit AA-Tempo / $O_2$ / Salpetersäure, bromfrei

Material:

| | | |
|---|---|---|
| 1-Propanol | 200 mmol | → 119,34 g/L |
| AA-TEMPO | 3.5 mol-% | → 14,94 g/L |
| Salpetersäure (> 99.8 %) | 3.5 mol-% | → 4,53 g/L |
| Essigsäure (100%) | 100 mL | |
| **logP 1-Propanol** | **0.55** | |

Reaktionsbedingungen:

| | |
|---|---|
| Reaktionstemperatur: | T = 50 °C |
| Reaktionszeit: | t = 3 h |
| Sauerstoff: | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50°C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 27 % beobachtet bei einer Selektivität für den Aldehyden (Propanal) von 20 %. Als Nebenprodukt kann Propylacetat in geringem Ausmaß detektiert werden.

Abbildung 5 zeigt die Entwicklung der 1-Propanol-, Aceton- und Isopropylacetat-Konzentrationen in Abhängigkeit von der Zeit bei der Durchführung des erfindungsgemäßen Verfahrens. Die Auswertung der Konzentrationen erfolgte über die Peakflächen.

3. Oxidation von Cyclohexanol mit AA-Tempo / $O_2$ / Salpetersäure, bromfrei

Material:

| | | |
|---|---|---|
| Cyclohexanol | 200 mmol | → 200,32 g/L |

(fortgesetzt)

| | | |
|---|---|---|
| AA-TEMPO | 3.5 mol-% | → 14,94 g/L |
| Salpetersäure (> 99.8 %) | 3.5 mol-% | → 4,53 g/L |
| Essigsäure (100%) | 100 mL | |
| **logP Cyclohexanol** | **1.27** | |

Reaktionsbedingungen:

| | |
|---|---|
| Reaktionstemperatur: | T = 50 °C |
| Reaktionszeit: | t = 3 h |
| Sauerstoff: | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50°C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 14 % beobachtet bei einer Selektivität für das Keton (Cyclohexanon) von 81 %. Als Nebenprodukt kann Cyclohexylacetat in geringem Ausmaß detektiert werden.

Abbildung 6 zeigt die Entwicklung der Cyclohexanol-, Cyclohexanon- und Cyclohexylacetat-Konzentrationen in Abhängigkeit von der Zeit bei der Durchführung des erfindungsgemäßen Verfahrens. Die Auswertung der Konzentrationen erfolgt über die Peakflächen.

4. Oxidation von Furfurylalkohol mit AA-Tempo / $O_2$ / Salpetersäure, bromfrei

Material:

| | | |
|---|---|---|
| Furfurylalkohol | 200 mmol | → 197,8 g/L |
| AA-TEMPO | 3.5 mol-% | → 14,94 g/L |
| Salpetersäure (> 99.8 %) | 3.5 mol-% | → 4,53 g/L |
| Essigsäure (100%) | 100 mL | |
| **logP Furfurylalkohol** | **0,08** | |

Reaktionsbedingungen:

| | |
|---|---|
| Reaktionstemperatur: | T = 50 °C |
| Reaktionszeit: | t = 3 h |
| Sauerstoff: | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50°C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 96 % beobachtet bei einer Selektivität für den Aldehyden (Furfural) von 93 %. Als Nebenprodukt kann Furfurylacetat in geringem Ausmaß detektiert werden.

Abbildung 7 zeigt die Entwicklung der Furfurylalkohol-, Furfural- und Furfurylacetat-Konzentrationen in Abhängigkeit von der Zeit bei der Durchführung des erfindungsgemäßen Verfahrens. Die Auswertung der Konzentrationen erfolgt über die Peakflächen.

5. Oxidation von 1,3-Dihydroxycyclohexan mit AA-Tempo / $O_2$ / Salpetersäure, bromfrei

Material:

| | | |
|---|---|---|
| 1,3-Dihydroxycyclohexan | 86 mmol | → 100 g/L |
| AA-TEMPO | 3.5 mol-% | → 6,49 g/L |
| Salpetersäure (> 99.8 %) | 3.5 mol-% | → 1,51 g/L |
| Essigsäure (100%) | 100 mL | |
| **logP 1,3-Dihydroxycyclohexan** | **0,01** | |

Reaktionsbedingungen:

| | |
|---|---|
| Reaktionstemperatur: | T = 50 °C |
| Reaktionszeit: | t = 3 h |
| Sauerstoff: | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50°C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 11 % beobachtet bei einer Selektivität für das monooxygenierte Keton (3-Hydroxycyclohexanol) von 72 %. Als Nebenprodukt kann 2-Cyclohexenon in geringem Ausmaß detektiert werden.

Abbildung 8 zeigt die Entwicklung der 1,3-Dihydroxycyclohexan-, 3-Hydroxycyclohexanon- und 2-Cyclohexenon-Konzentrationen in Abhängigkeit von der Zeit bei der Durchführung des erfindungsgemäßen Verfahrens. Die Auswertung der Konzentrationen erfolgt über die Peakflächen.

6. Oxidation von Nicotinylalkohol mit AA-Tempo / $O_2$ / Salpetersäure, bromfrei

Material:

| | | |
|---|---|---|
| Nicotinylalkohol | 200 mmol | → 218,26 g/L |
| AA-TEMPO | 3.5 mol-% | → 14,94 g/L |
| Salpetersäure (> 99.8 %) | 3.5 mol-% | → 4,53 g/L |
| Essigsäure (100%) | 100 mL | |
| **logP Nicotinylalkohol** | **0,12** | |

Reaktionsbedingungen:

| | |
|---|---|
| Reaktionstemperatur: | T = 50 °C |
| Reaktionszeit: | t = 3 h |
| Sauerstoff: | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50 °C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 17 % beobachtet bei einer Selektivität für den Aldehyden (Niotinaldehyd) von 79 %. Als Nebenprodukt kann Nicotinylacetat in geringem Ausmaß detektiert werden.

Abbildung 9 zeigt die Entwicklung der Nicotinylalkohol-, Nicotinaldehyd- und Nicotinylacetat-Konzentrationen in Abhängigkeit von der Zeit bei der Durchführung des erfindungsgemäßen Verfahrens. Die Auswertung der Konzentrationen erfolgt über die Peakflächen.

7. Oxidation von Borneol mit AA-Tempo / $O_2$ / Salpetersäure, bromfrei

Material:

| | | |
|---|---|---|
| Borneol | 200 mmol | → 308,50 g/L |
| AA-TEMPO | 3.5 mol-% | → 14,94 g/L |
| Salpetersäure (> 99.8 %) | 3.5 mol-% | → 4,53 g/L |
| Essigsäure (100%) | 100 mL | |
| **logP Borneol** | **1,63** | |

Reaktionsbedingungen:

| | |
|---|---|
| Reaktionstemperatur: | T = 50 °C |

(fortgesetzt)

| | |
|---|---|
| Reaktionszeit: | t = 3 h |
| Sauerstoff : | 1 bar |
| Reaktorvolumen | 250 mL |

Durchführung:

Das Edukt wird vorgelegt, in der Essigsäure (Zugabe der Essigsäure bis das Volumen von 100 ml erreicht ist) gelöst und in den Reaktor überführt. Anschließend wird die Lösung unter Rühren und Sauerstoffbegasung auf eine Temperatur von 50°C aufgeheizt. Zum Start der Reaktion werden der Reaktionsmischung die Säure und der Katalysator hinzugefügt. Der Druck in dem Reaktor wird bei 1 bar konstant gehalten, wobei verbrauchter Sauerstoff durch eine geöffnete Sauerstoffversorgung bis zu einem Druck von 1 bar nachdosiert wird. Nach drei Stunden wird die Reaktion durch Unterbrechen der Sauerstoffzufuhr und Abkühlung des Systems abgebrochen.

Ergebnis:

Es wird ein Umsatz von größer 22 % beobachtet bei einer Selektivität für das Keton (Menthol) von 82,9 %. Als Nebenprodukt kann Bornylacetat in geringem Ausmaß detektiert werden.

Abbildung 10 zeigt die Entwicklung der Borneol-, Menthol- und Bornylacetat-Konzentrationen in Abhängigkeit von der Zeit bei der Durchführung des erfindungsgemäßen Verfahrens. Die Auswertung der Konzentrationen erfolgt über die Peakflächen.

## Patentansprüche

1. Verfahren zur Herstellung von Aldehyden und Ketonen umfassend die Oxidation von primären oder sekundären Alkoholen mit einem sauerstoffhaltigen Gas in Gegenwart einer Katalysatorzusammensetzung umfassend mindestens ein Nitroxylradikal, eine oder mehrere NO-Quellen und mindestens eine oder mehrere Carbonsäuren oder deren Anhydride oder eine oder mehrere Carbonsäuren oder deren Anhydride und Mineralsäuren oder deren Anhydride, ggf. in Gegenwart eines oder mehrerer Lösungsmittel, **dadurch gekennzeichnet, dass** die eingesetzten primären und sekundären Alkohole einen Wert für den dekadischen Logarithmus des n-Oktanol-Wasser-Verteilungskoeffizienten (log P) von weniger als 2 aufweisen.

2. Verfahren gemäß Anspruch 1, **dadurch gekennzeichnet, dass** die Aldehyde und Ketone mit einer Ausbeute von mehr als 92 %, bezogen auf den eingesetzten Alkohol, erhalten werden.

3. Verfahren gemäß Anspruch 1 oder 2, **dadurch gekennzeichnet, dass** als Nitroxylradikale 2,2,6,6-Tetramethylpiperidin-1-oxyl (TEMPO) und/oder die an Position 4 des Heterocyclus substituierten Derivate des 2,2,6,6-Tetramethylpiperidin-1-oxyls eingesetzt werden, wobei die Derivate einen oder mehrere Substituenten ausgewählt aus $R^1$, $C_1$-$C_8$-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen aufweisen, worin $R^1$ eine ($C_1$-$C_{10}$)-Alkyl-, ($C_1$-$C_{10}$)-Alkenyl-, ($C_1$-$C_{10}$)-Alkoxy-, ($C_6$-$C_{18}$)-Aryl-, ($C_7$-$C_{19}$)-Aralkyl-, ($C_6$-$C_{18}$)-Aryl-($C_1$-$C_8$)-Alkyl- oder ($C_3$-$C_{18}$)-Heteroarylgruppe bedeutet.

4. Verfahren gemäß einem oder mehreren der Ansprüche 1 bis 3, **dadurch gekennzeichnet, dass** der Anteil an Nitroxylradikal 0,001 bis 20 mol-%, bezogen auf die Menge des eingesetzten Alkohols, beträgt.

5. Verfahren gemäß einem oder mehreren der Ansprüche 1 bis 4, **dadurch gekennzeichnet, dass** als Carbonsäure Essigsäure oder als Anhydrid Essigsäureanhydrid eingesetzt wird.

6. Verfahren gemäß einem oder mehreren der Ansprüche 1 bis 5, **dadurch gekennzeichnet, dass** der Anteil an Carbonsäure und/oder des Anhydrids 0,1 bis 200 mol-%, bezogen auf die Menge an eingesetztem Alkohol beträgt.

7. Verfahren gemäß einem oder mehreren der Ansprüche 1 bis 6, **dadurch gekennzeichnet, dass** als Mineralsäuren $H_2CO_3$, $H_3PO_4$, $H_2SO_4$, $H_2SO_3$, $H_3BO_3$ oder deren Anhydride oder Mischungen hieraus eingesetzt werden.

8. Verfahren gemäß einem oder mehreren der Ansprüche 1 bis 7, **dadurch gekennzeichnet, dass** als NO-Quelle Ammoniumnitrat oder-nitrit, Alkali- oder Erdalkalinitrate oder -nitrite, stickoxidhaltige Gase oder Mischungen hieraus eingesetzt werden.

9. Verfahren gemäß einem oder mehreren der Ansprüche 1 bis 8, **dadurch gekennzeichnet, dass** der Anteil der eingesetzten NO-Quelle(n) 0,001 bis 10 mol-%, bezogen auf die Menge an eingesetztem Alkohol, beträgt.

10. Verfahren gemäß einem oder mehreren der Ansprüche 1 bis 9, **dadurch gekennzeichnet, dass** kein zusätzliches Lösungsmittel eingesetzt wird.

11. Verfahren gemäß einem oder mehreren der Ansprüche 1 bis 10, **dadurch gekennzeichnet, dass** das Reaktionswasser aus dem Reaktionsgemisch entfernt wird.

12. Verfahren gemäß Anspruch 11, **dadurch gekennzeichnet, dass** die Entfernung des Reaktionswassers durch Zusatz wasserabsorbierender Mittel zur Reaktionsmischung und/oder durch destillative oder extraktive Entfernung aus der Reaktionsmischung während der Reaktion erfolgt.

13. Verfahren gemäß Anspruch 11 oder 12, **dadurch gekennzeichnet, dass** Wasser komplexierende Lösungsmittel oder Lösungsmittel, die Wasser chemisch binden, eingesetzt werden.

14. Verfahren gemäß einem oder mehreren der Ansprüche 1 bis 13, **dadurch gekennzeichnet, dass** in Folgeschritten die Carbonsäure und ggf. das Lösungsmittel durch Destillation oder Extraktion entfernt wird, die Nitroxylradikale extrahiert und ggf. rezykliert werden, ggf. die aus den NO-Quellen stammenden Salze entfernt werden und/oder das Aldehyd bzw. Keton durch Kristallisation, Destillation, Extraktion und/oder chromatographische Trennung gereinigt wird.

**Claims**

1. A process for producing aldehydes and ketones comprising the oxidation of primary or secondary alcohols with an oxygen-containing gas in the presence of a catalyst composition comprising at least one nitroxyl radical, one or more NO sources and at least one or more carboxylic acids or anhydrides or one or more carboxylic acids or anhydrides and mineral acids or anhydrides, optionally in the presence of one or more solvents, **characterized in that** the primary and secondary alcohols used have a value of less than 2 for the decadic logarithm of the n-octanol-water partition coefficient (logP).

2. The process according to claim 1, **characterized in that** the aldehydes and ketones are obtained in a yield of more than 92%, based on the alcohol used.

3. The process according to claim 1 or 2, **characterized in that** the one or more nitroxyl radicals used comprise 2,2,6,6-tetramethyl-piperidine-1-oxyl (TEMPO) and/or the 2,2,6,6-tetramethylpiperidine-1-oxyl derivatives substituted at position 4 of the heterocycle, wherein the derivatives display one or more substituents selected from $R^1$, $C_1$-$C_8$-amido, halogen, oxy, hydroxyl, amino, alkylamino, dialkylamino, arylamino, diarylamino, alkylcarbonyloxy, arylcarbonyloxy, alkylcarbonylamino and arylcarbonylamino groups, where $R^1$ is a ($C_1$-$C_{10}$)-alkyl, ($C_1$-$C_{10}$)-alkenyl, ($C_1$-$C_{10}$)-alkoxy, ($C_6$-$C_{18}$)-aryl, ($C_7$-$C_{19}$)-aralkyl, ($C_6$-$C_{18}$)-aryl-($C_1$-$C_8$)-alkyl or ($C_3$-$C_{18}$) -heteroaryl group.

4. The process according to one or more of claims 1 to 3, **characterized in that** the proportion of nitroxyl radical is in the range from 0.001 to 20 mol%, based on the amount of alcohol used.

5. The process according to one or more of claims 1 to 4, **characterized in that** acetic acid is used as carboxylic acid or acetic anhydride is used as anhydride.

6. The process according to one or more of claims 1 to 5, **characterized in that** the proportion of carboxylic acid and/or anhydride is in the range from 0.1 to 200 mol%, based on the amount of alcohol used.

7. The process according to one or more of claims 1 to 6, **characterized in that** $H_2CO_3$, $H_3PO_4$, $H_2SO_4$, $H_2SO_3$, $H_3BO_3$ or their anhydrides or mixtures thereof are used as mineral acids.

8. The process according to one or more of claims 1 to 7, **characterized in that** the one or more NO sources used comprise ammonium nitrate or nitrite, alkali or alkaline earth metal nitrates or nitrites, nitrous gases or mixtures thereof.

9. The process according to one or more of claims 1 to 8, **characterized in that** the proportion of NO source(s) used is in the range from 0.001 to 10 mol%, based on the amount of alcohol used.

10. The process according to one or more of claims 1 to 9, **characterized in that** no additional solvent is used.

11. The process according to one or more of claims 1 to 10, **characterized in that** the water of reaction is removed from the reaction mixture.

12. The process according to claim 11, **characterized in that** the water of reaction is removed by addition of water-absorbing agents to the reaction mixture and/or by distillative or extractive removal from the reaction mixture during the reaction.

13. The process according to claim 11 or 12, **characterized in that** water-complexing solvents or solvents that bind water chemically are used.

14. The process according to one or more of claims 1 to 13, **characterized in that** subsequent steps comprise the carboxylic acid and, if used, the solvent being removed by distillation or extraction, the nitroxyl radicals being extracted and optionally recycled, any salts from the NO sources being removed and/or the aldehyde/ketone being purified by crystallization, distillation, extraction and/or chromatographic separation.

**Revendications**

1. Procédé pour la préparation d'aldéhydes et de cétones comprenant l'oxydation d'alcools primaires ou secondaires avec un gaz contenant de l'oxygène en présence d'une composition catalytique comprenant au moins un radical nitroxyle, une ou plusieurs sources de NO et au moins un ou plusieurs acides carboxyliques ou leurs anhydrides ou un ou plusieurs acides carboxyliques ou leurs anhydrides et des acides minéraux ou leurs anhydrides, le cas échéant en présence d'un ou de plusieurs solvants, **caractérisé en ce que** les alcools primaires et secondaires utilisés présentent une valeur pour le logarithme décimal du coefficient de répartition n-octanol-eau (log P) inférieure à 2.

2. Procédé selon la revendication 1, **caractérisé en ce que** les aldéhydes et les cétones sont obtenus avec un rendement supérieur à 92%, par rapport à l'alcool utilisé.

3. Procédé selon la revendication 1 ou 2, **caractérisé en ce qu'**on utilise, comme radicaux nitroxyle, le 2,2,6,6-tétraméthylpipéridin-1-oxyle (TEMPO) et/ou les dérivés substitués en position 4 de l'hétérocycle du 2,2,6,6-tétraméthylpipéridin-1-oxyle, les dérivés présentant un ou plusieurs substituants choisis parmi $R^1$, des groupes $C_1$-$C_8$-amido, halogéno, oxy, hydroxy, amino, alkylamino, dialkylamino, arylamino, diarylamino, alkylcarbonyloxy, arylcarbonyloxy, alkylcarbonylamino et arylcarbonylamino, où $R^1$ représente un groupe ($C_1$-$C_{10}$)-alkyle, ($C_1$-$C_{10}$) -alcényle, ($C_1$-$C_{10}$) -alcoxy, ($C_6$-$C_{18}$)-aryle, ($C_7$-$C_{19}$) -aralkyle, ($C_6$-$C_{18}$) -aryl- ($C_1$-$C_8$) -alkyle ou ($C_3$-$C_{18}$) -hétéroaryle.

4. Procédé selon l'une ou plusieurs des revendications 1 à 3, **caractérisé en ce que** la proportion de radical nitroxyle représente 0,001 à 20% en mole, par rapport à la quantité de l'alcool utilisé.

5. Procédé selon l'une ou plusieurs des revendications 1 à 4, **caractérisé en ce qu'**on utilise, comme acide carboxylique, de l'acide acétique ou, comme anhydride, de l'anhydride de l'acide acétique.

6. Procédé selon l'une ou plusieurs des revendications 1 à 5, **caractérisé en ce que** la proportion d'acide carboxylique et/ou d'anhydride représente 0,1 à 200% en mole, par rapport à la quantité de l'alcool utilisé.

7. Procédé selon l'une ou plusieurs des revendications 1 à 6, **caractérisé en ce qu'**on utilise, comme acides minéraux, $H_2CO_3$, $H_3PO_4$, $H_2SO_4$, $H_2SO_3$, $H_3BO_3$ ou leurs anhydrides ou des mélanges de ceux-ci.

8. Procédé selon l'une ou plusieurs des revendications 1 à 7, **caractérisé en ce qu'**on utilise, comme source de NO,

du nitrate ou du nitrite d'ammonium, des nitrates ou des nitrites de métal alcalin ou de métal alcalino-terreux, des gaz contenant de l'oxyde d'azote ou des mélanges de ceux-ci.

**9.** Procédé selon l'une ou plusieurs des revendications 1 à 8, **caractérisé en ce que** la proportion de la/des source(s) de NO utilisée(s) représente 0,001 à 10% en mole, par rapport à la quantité de l'alcool utilisé.

**10.** Procédé selon l'une ou plusieurs des revendications 1 à 9, **caractérisé en ce qu'**on n'utilise pas de solvant supplémentaire.

**11.** Procédé selon l'une ou plusieurs des revendications 1 à 10, **caractérisé en ce que** l'eau de réaction est éliminée du mélange réactionnel.

**12.** Procédé selon la revendication 11, **caractérisé en ce que** l'élimination de l'eau de réaction est réalisée par addition d'agents absorbant l'eau au mélange réactionnel et/ou par élimination par distillation ou par extraction du mélange réactionnel pendant la réaction.

**13.** Procédé selon la revendication 11 ou 12, **caractérisé en ce qu'**on utilise des solvants qui complexent l'eau ou des solvants qui lient l'eau chimiquement.

**14.** Procédé selon l'une ou plusieurs des revendications 1 à 13, **caractérisé en ce que** dans des étapes consécutives, l'acide carboxylique et le cas échéant le solvant sont éliminés par distillation ou extraction, les radicaux nitroxyle sont extraits et le cas échéant recyclés, le cas échéant les sels provenant des sources de NO sont éliminés et/ou l'aldéhyde ou la cétone est purifié(e) par cristallisation, distillation, extraction et/ou séparation chromatographique.

Abb. 1: Oxidation von Isosorbit mit AA-Tempo / Nitrit / Nitrat / Sauerstoff

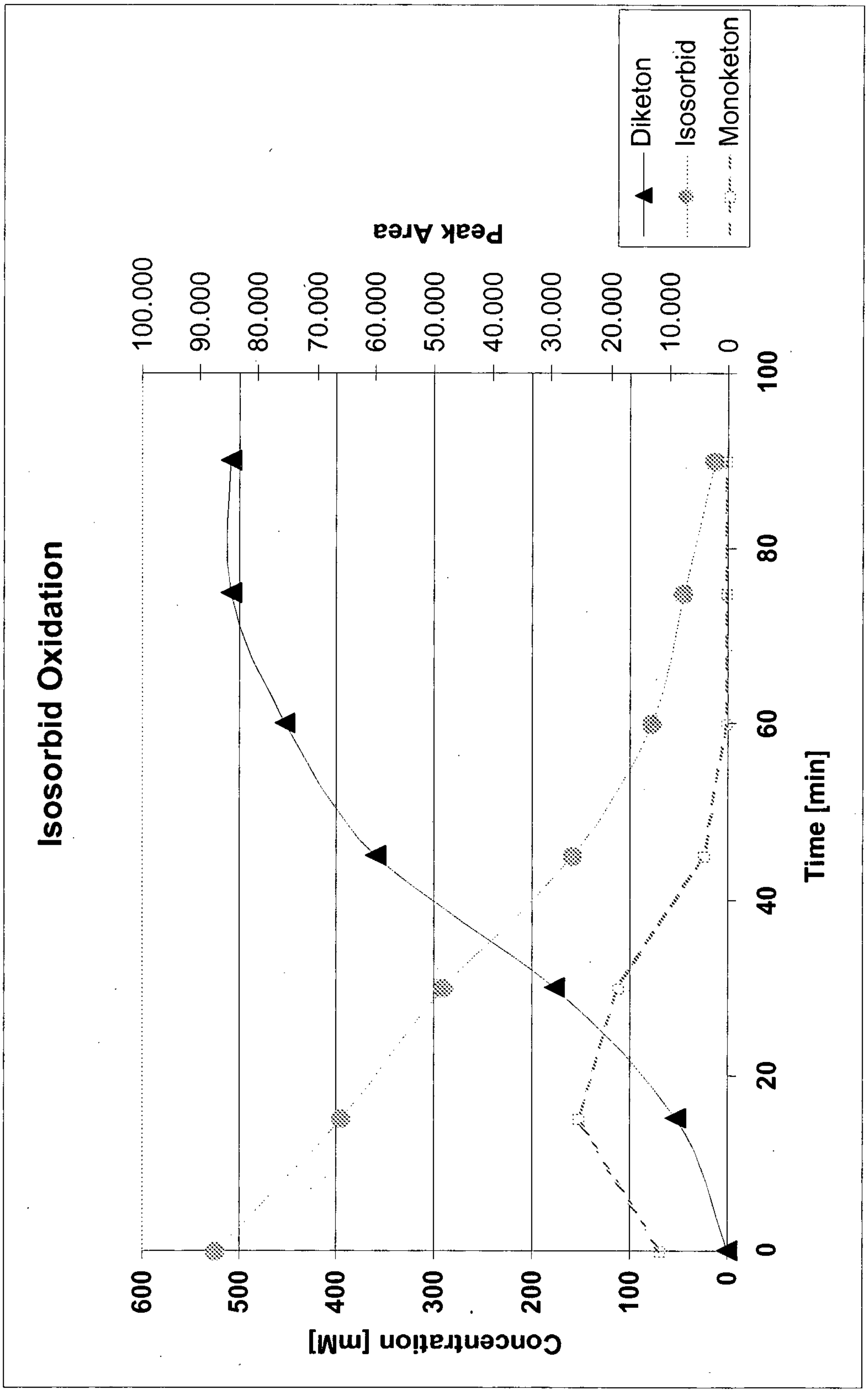

Abb. 2: Oxidation von Isomannit mit AA-Tempo / Nitrit / Nitrat / Sauerstoff

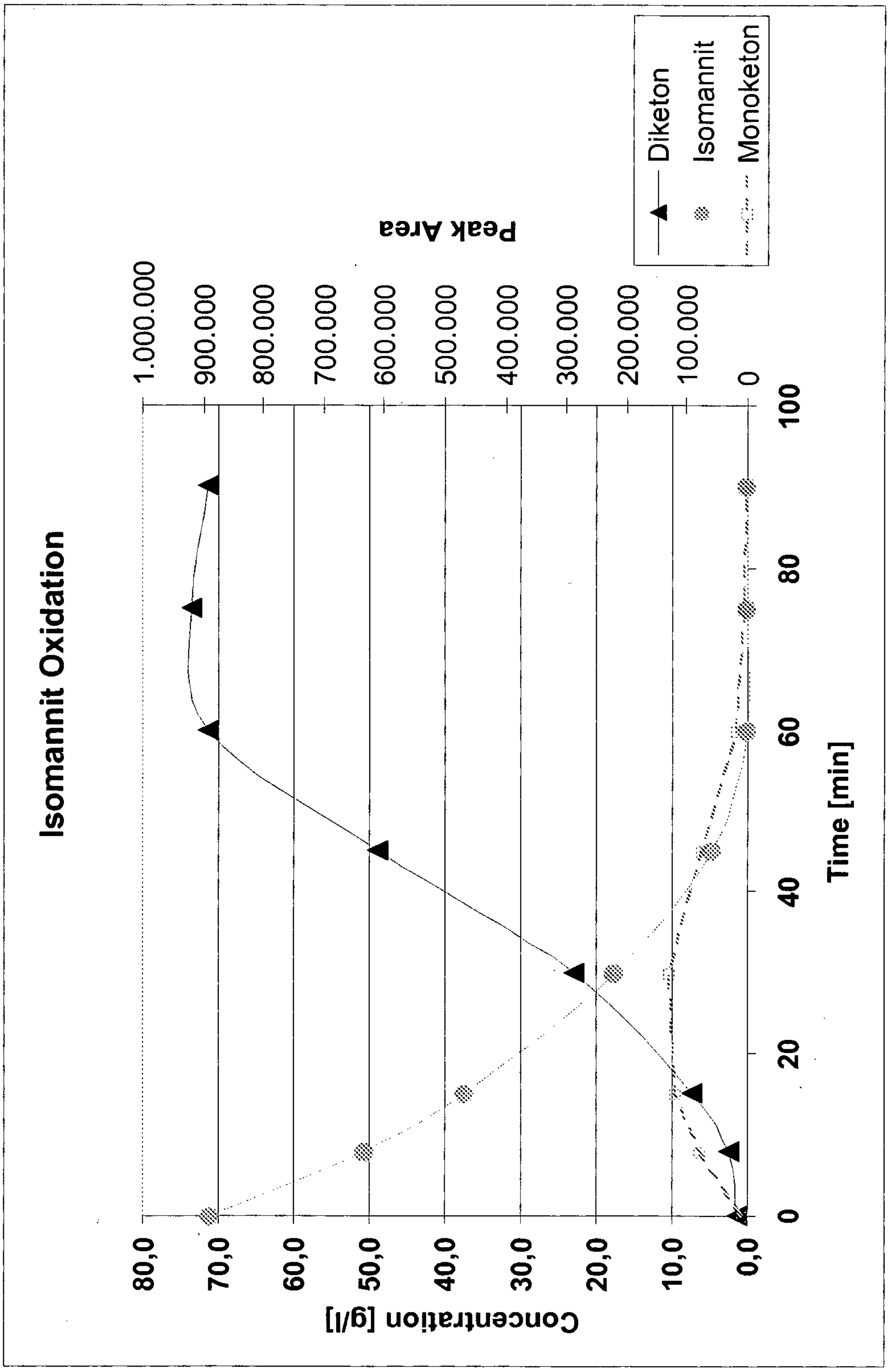

Abb. 3

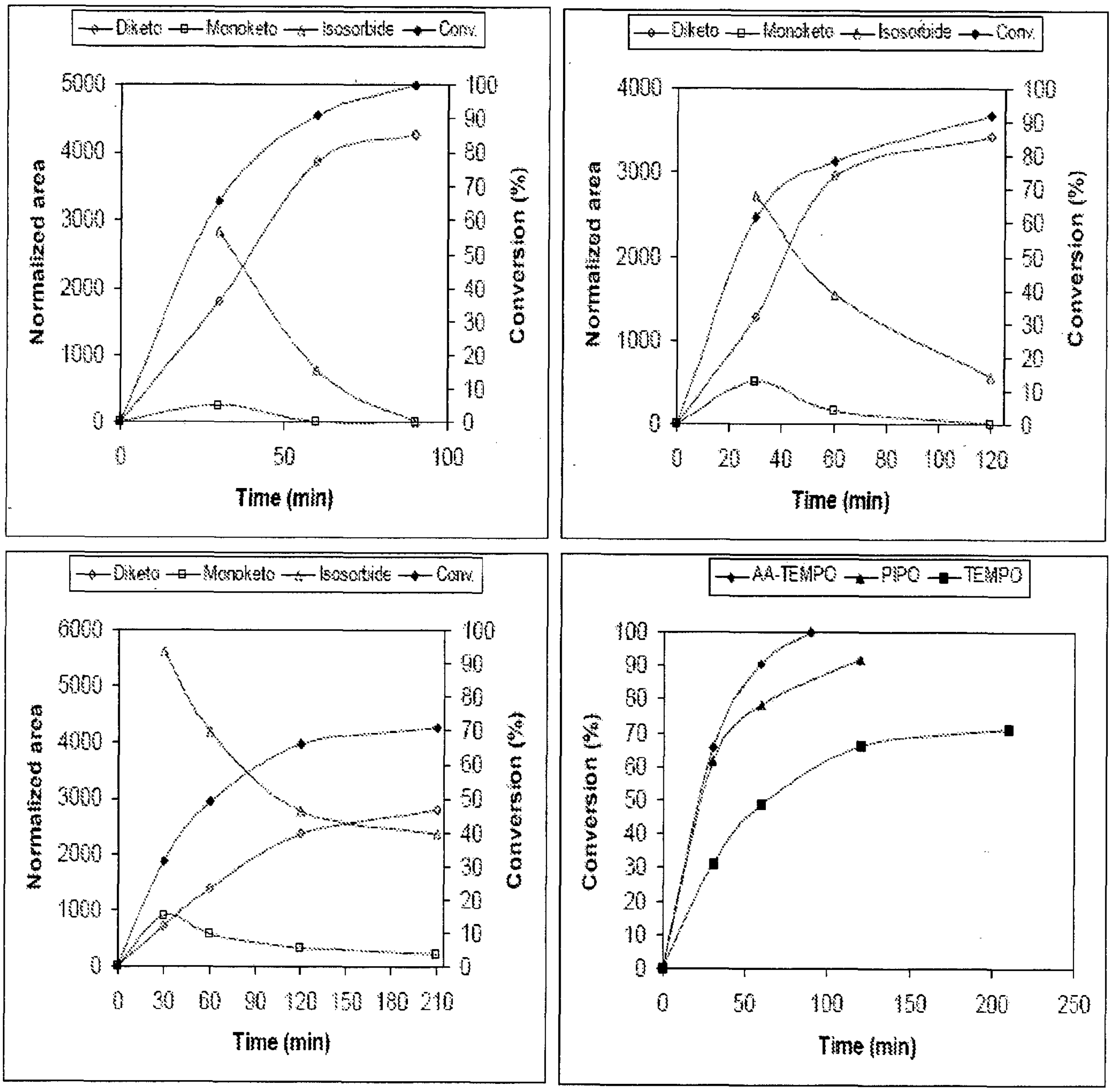
Diketo
Monoketo
Isosorbide
Conv.
Normalized area
Conversion (%)
Time (min)
AA-TEMPO
PIPO
TEMPO

Abbildung 4: Oxidation von 2-Propanol mit AA-TEMPO / Salpetersäure / Sauerstoff

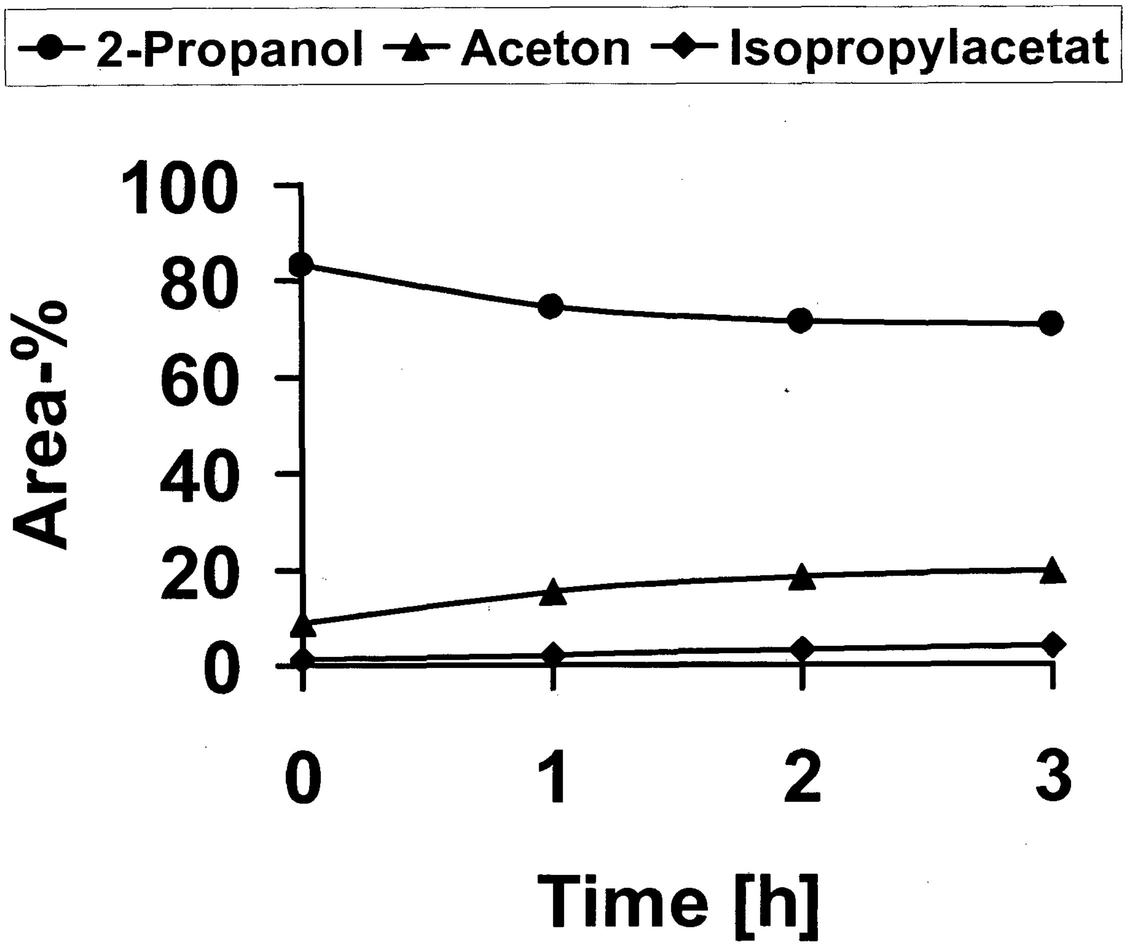

Abbildung 5: Oxidation von 1-Propanol mit AA-TEMPO / Salpetersäure / Sauerstoff

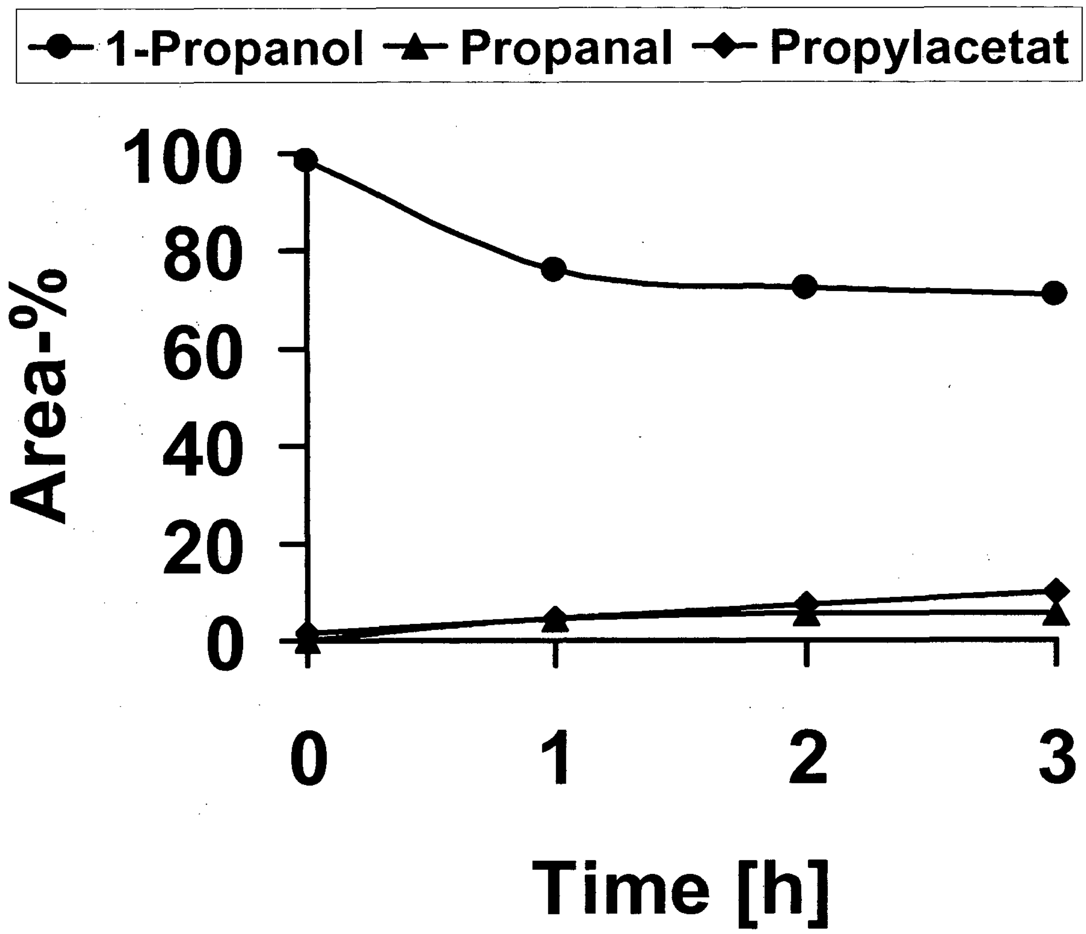

Abbildung 6: Oxidation von Cyclohexanol mit AA-TEMPO / Salpetersäure / Sauerstoff

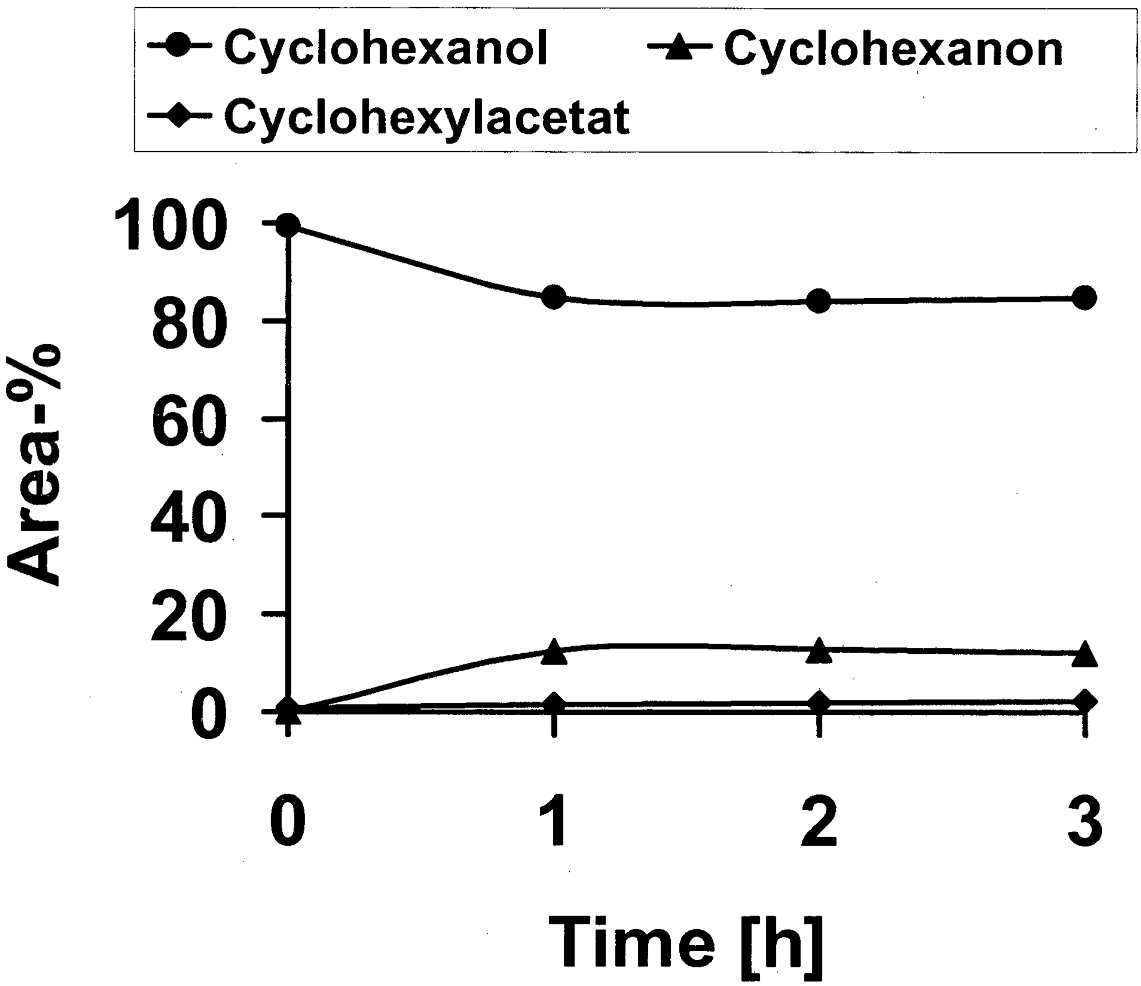

Abbildung 7: Oxidation von Furfurylalkohol mit AA-TEMPO / Salpetersäure / Sauerstoff

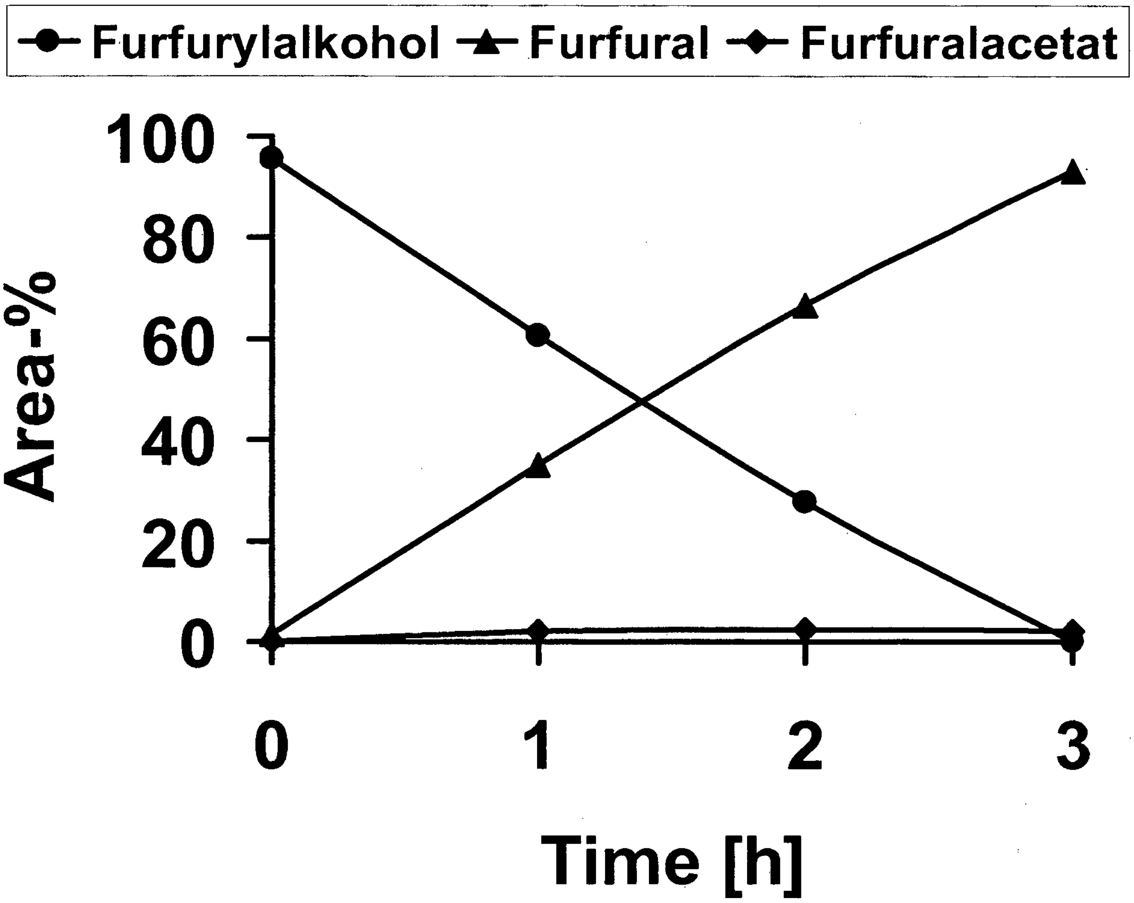

Abbildung 8: Oxidation von 1,3-Dihydroxycyclohexan mit AA-TEMPO / Salpetersäure / Sauerstoff

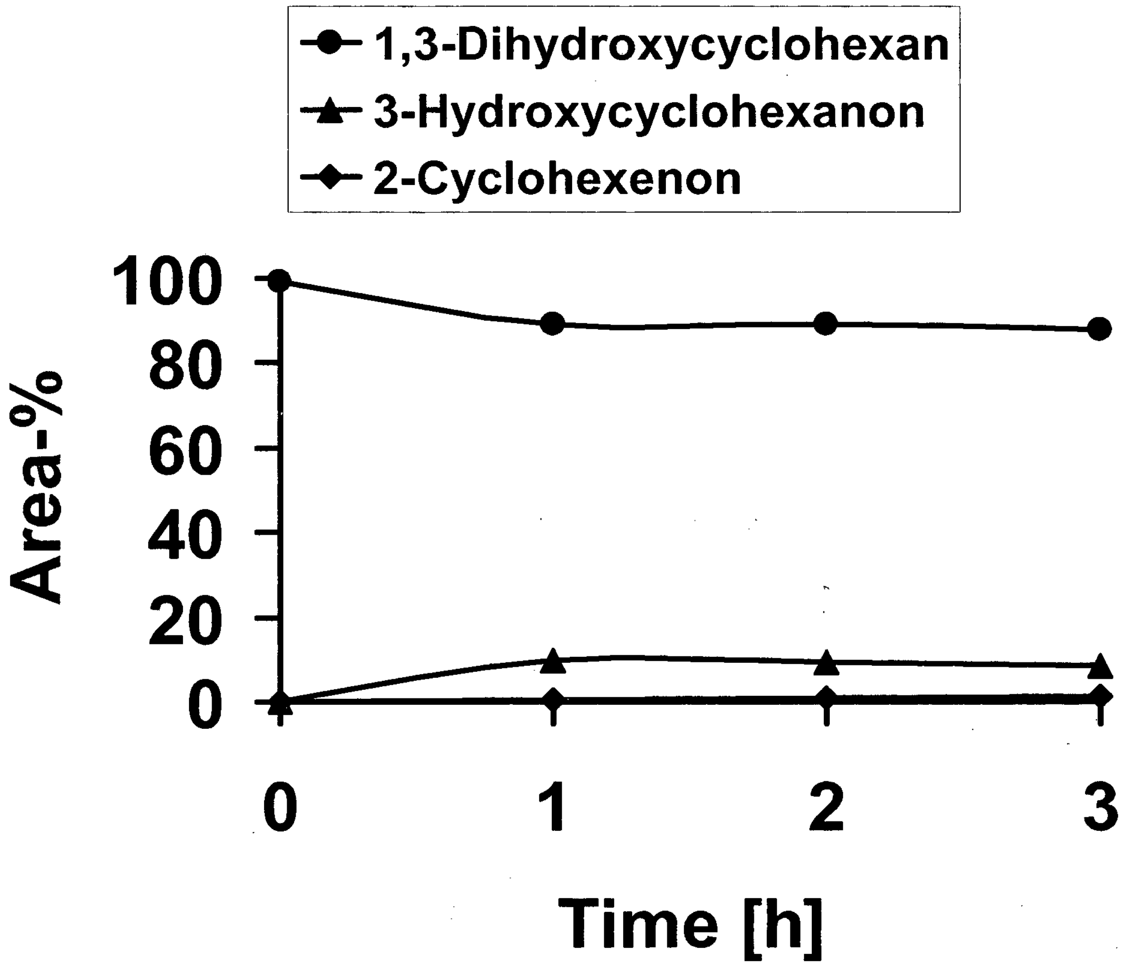

Abbildung 9: Oxidation von Nicotinylalkohol mit AA-TEMPO / Salpetersäure / Sauerstoff

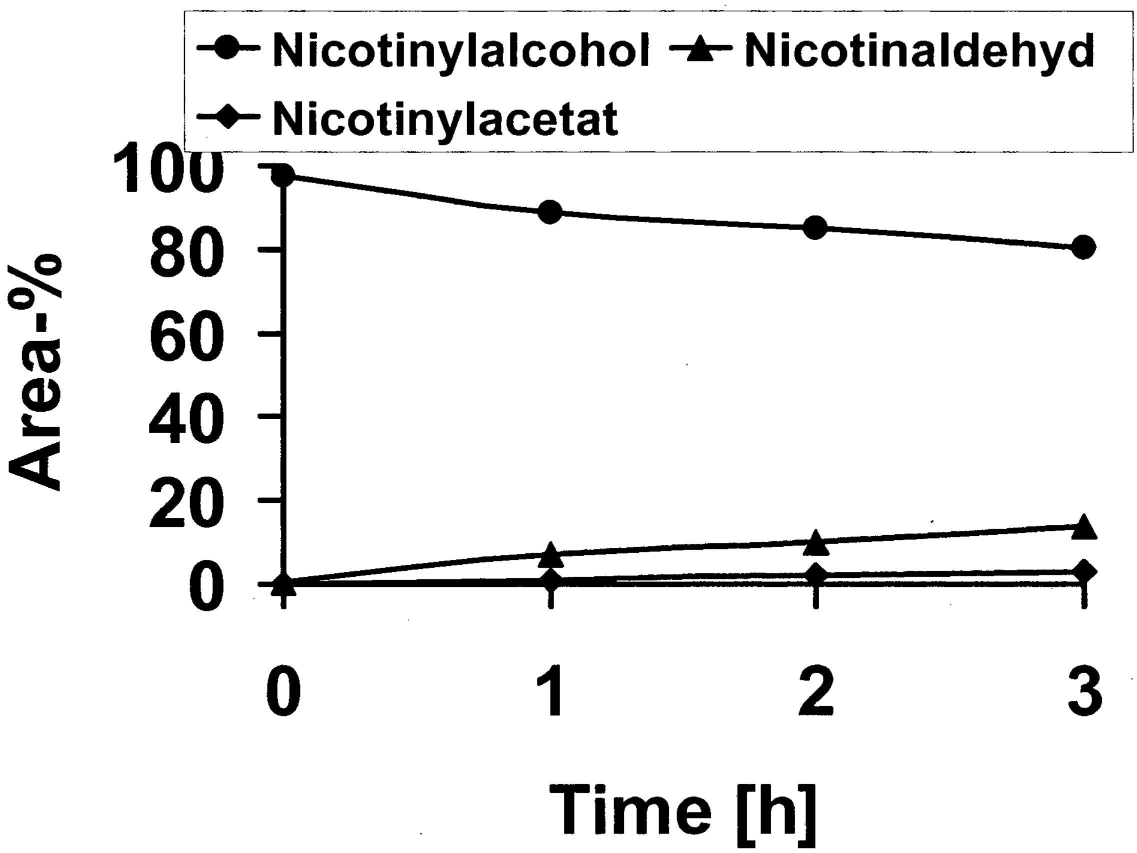

Abbildung 10: Oxidation von Borneol mit AA-TEMPO / Salpetersäure / Sauerstoff

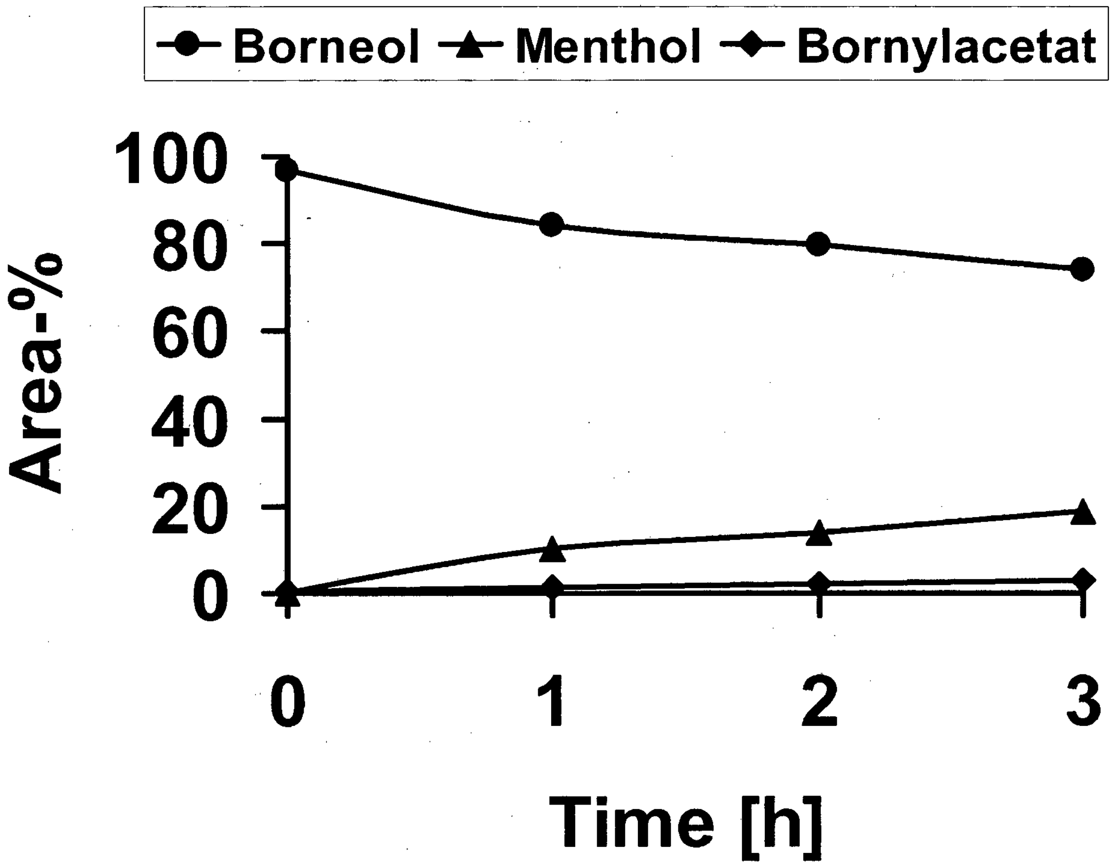

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 7030279 B
- US 5136103 A
- US 5155280 A
- US 5239116 A
- US 5155279 A
- US 5336811 A


### In der Beschreibung aufgeführte Nicht-Patentliteratur


- **A.K. HOFFMANN ; A.T. HENDERSON.** *J. Am. Chem. Soc.,* 1961, vol. 83, 4671
- **O.L. LEBEDER ; S.N. KAZARNOVSKII.** *Zh. Obshch. Khim.,* 1960, vol. 30, 1631
- **O.L. LEBEDER ; S.N. KAZARNOVSKII.** *CA,* 1961, vol. 55, 1473a
- **J.M. BOBBITT ; C.L. FLORES.** *Heterocycles,* 1988, vol. 27, 509
- **A.E.J. DE NOOY ; A.C. BESEMER ; H. VAN BEKKUM.** *Synthesis,* 1996, 1153
- **L. ANELLI ; C. BIFFI ; F. MONTANARI ; S. QUICI.** *J. Org. Chem.,* 1987, vol. 52, 2559
- **J.A. CELLA ; J.A. KELLEY ; E.F. KENEHAN.** *J. Org. Chem.,* 1975, vol. 40, 1850
- **S.D. RYCHOVSKY ; R. VAIDYANATHAN.** *J. Org. Chem.,* 1999, vol. 64, 310
- **BOLM, CARSTEN ; MAGNUS, ANGELIKA S. ; HILDEBRAND, JENS P.** *Organic Letters,* 2000, vol. 2 (8), 1173-1175
- **S.S. KIM ; K. NEHRU.** *Synletter,* 2002, 616
- **DE LUCA, LIDIA ; GIACOMELLI, GIAMPAOLO ; PORCHEDDU, ANDREA.** *Organic Letters,* 2001, vol. 3 (19), 3041-3043
- **SHELDON, ROGER A. ; ARENDS, ISABEL W. C. E.** *Journal of Molecular Catalysis A: Chemic,* 2006, vol. 251 (1-2), 200-214
- **MINISCI, FRANCESCO ; PUNTA, CARLO ; RECUPERO, FRANCESCO.** *Journal of Molecular Catalysis A: Chemical,* 2006, vol. 251 (1-2), 129-149
- **XINQUAN HU et al.** *J. AM. CHEM. SOC.,* 2004, vol. 126, 4112-4113
- Octanol-Water Partition Coefficients: Fundamentals and Physical Chemistry. **J. SANGSTER.** Wiley Series in Solution Chemistry. John Wiley & Sons, 1997, vol. 2
- *Eur J Med Chem.,* Juli 2000, vol. 35 (7-8), 651-61
- **GUDRUN WIENKE.** Messung und Vorausberechnung von n-Octanol/Wasser-Verteilungskoeffizienten. *Doktorarbeit, Univ. Oldenburg,* 1993, 1-172